# EUROPÄISCHE PATENTSCHRIFT

(11) **EP 0 729 460 B1**
(45) Veröffentlichungstag und Bekanntmachung des Hinweises auf die Patenterteilung: **15.05.2002**
(21) Anmeldenummer: 94930215.2
(22) Anmeldetag: 24.10.1994
(51) Int. Cl.: C07D 233/76, A61K 31/415, C07D 401/12

(54) **SUBSTITUIERTE 5-RING-HETEROCYCLEN, IHRE HERSTELLUNG UND IHRE VERWENDUNG**
SUBSTITUTED 5-RING HETEROCYCLES, THEIR PREPARATION AND THEIR USE
HETEROCYCLES SUBSTITUES A 5 NOYAUX, LEUR PREPARATION ET LEUR UTILISATION

(30) Priorität: 15.11.1993 DE 4338944; 08.08.1994 DE 4427979
(43) Veröffentlichungstag der Anmeldung: 04.09.1996
(73) Patentinhaber: HOECHST AKTIENGESELLSCHAFT, 65929 Frankfurt am Main (DE)
(72) Erfinder: ZOLLER, Gerhard, D-61137 Schöneck (DE); KLINGLER, Otmar, D-63110 Rodgau (DE); JABLONKA, Bernd, D-65812 Bad Soden (DE); JUST, Melitta, D-63225 Langen (DE); BREIPOHL, Gerhard, D-60529 Frankfurt am Main (DE); KNOLLE, Jochen, D-65830 Kriftel (DE); KÖNIG, Wolfgang, D-94375 Stallwang (DE); STILZ, Hans-Ulrich, D-60529 Frankfurt am Main (DE)
(86) Internationale Anmeldenummer: EP9403491
(87) Internationale Veröffentlichungsnummer: WO9514008

(56) Entgegenhaltungen:
- EP-A- 0 006 352
- EP-A- 0 449 079
- EP-A- 0 512 831
- EP-A- 0 530 505
- EP-A- 0 566 919
- WO-A-93/18057
- GB-A- 2 032 419

## Beschreibung

Die vorliegende Erfindung betrifft substituierte 5-Ring-Heterocyclen, ihre Herstellung und ihre Verwendung als Heilmittel, insbesondere als Hemmstoffe der Blutplättchenaggregation.

In der EP-A-449 079, der EP-A-530 505, der EP-A-566 919 und der WO-A-93/18057 sind Hydantoinderivate beschrieben, die thrombozytenaggregationshemmende Wirkungen aufweisen. Die EP-A 512 831 erwähnt Pyrrolidon-Derivate, die die Fibrinogenbindung an Blutplättchen und dadurch die Aggregation der Plättchen verhindern. Weitere Untersuchungen zeigten, daß auch die Verbindungen der vorliegenden Erfindung starke Hemmstoffe der Blutplättchenaggregation sind.

Gegenstand der vorliegenden Erfindung sind 5-Ring-Heterocyclen der allgemeinen Formel I, 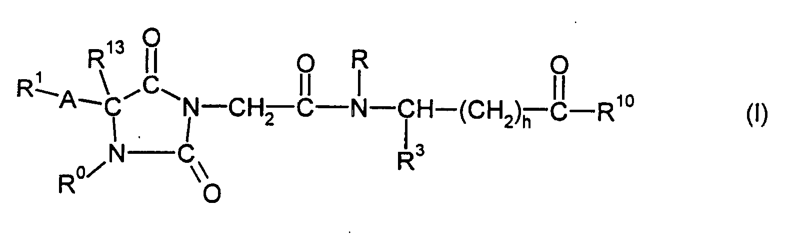 worin gleichzeitig
- A: für einen 1,4-Phenylenrest steht;
- R und R⁰: unabhängig voneinander für Wasserstoff oder (C₁-C₄)-Alkyl stehen;
- R¹: für H₂N-C(=NH), H₂N-C(=NH)-NH oder H₂N-CH₂ steht;
- R³: für den Rest CONHR¹⁴ steht, für den Rest CONHR¹⁵ steht oder für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht;
- R¹⁰: für Hydroxy oder (C₁-C₈)-Alkoxy steht;
- R¹³: für (C₁-C₆)-Alkyl, (C₃-C₇)-Cycloalkyl oder Benzyl steht;
- R¹⁴: für Methyl steht, das durch Phenyl und Hydroxycarbonyl substituiert ist, oder für Methyl steht, das durch Phenyl und (C₁-C₈)-Alkoxycarbonyl substituiert ist;
- R¹⁵: für einen Adamantylrest oder einen Adamantylmethylrest steht;
- h: für 1 oder 2 steht;
und deren physiologisch verträgliche Salze.

Cycloalkylreste sind insbesondere Cyclopropyl, Cyclobutyl, Cyclopentyl, Cyclohexyl, Cycloheptyl, die aber auch durch beispielsweise (C₁-C₄)-Alkyl substituiert sein können. Beispiele für substituierte Cycloalkylreste sind 4-Methylcyclohexyl und 2,3-Dimethylcyclopentyl.

Alkylreste können geradkettig oder verzweigt sein. Dies gilt auch, wenn sie Substituenten tragen oder als Substituenten anderer Reste auftreten, beispielsweise in Alkoxy- oder Alkoxycarbonylresten. Beispiele für geeignete Alkylreste sind: Methyl, Ethyl, Propyl, Butyl, Pentyl, Hexyl, Heptyl, Octyl, Isopropyl, Isopentyl, Neopentyl, Isohexyl, 3-Methylpentyl, sec.-Butyl, tert.-Butyl, tert.-Pentyl. Bevorzugte Alkylreste sind Methyl, Ethyl, Propyl, Isopropyl, Butyl, Isobutyl, sec.-Butyl und tert.-Butyl.

Auch Alkenylreste sowie Alkinylreste können geradkettig und verzweigt sein. Beispiele für Alkenylreste sind Vinyl, 1-Propenyl, Allyl, Butenyl, für Alkinylreste Ethinyl, 1-Propinyl oder Propargyl. Adamantylreste sind Beispiele von tricyclischen Resten, die unsubstituiert sein oder in beliebigen geeigneten Positionen durch eine oder mehrere Oxogruppen und/oder eine oder mehrere gleiche oder verschiedene (C₁-C₄)-Alkylguppen, z. B. Methyl- oder Isopropylgruppen, bevorzugt Methylgruppen, substituiert sein können und in denen sich die freie Bindung in einer beliebigen Position des Moleküls befinden kann. Beispiele für Adamantylreste sind der 1-Adamantylrest und der 2-Adamantylrest.

Phenyl, Naphthyl und Biphenylyl sind Beispiele von Arylgruppen, wobei 1-Naphthyl,
2-Naphthyl und insbesondere Phenyl bevorzugt sind. Arylreste, insbesondere Phenylreste, können ein- oder mehrfach, das heißt ein-, zwei- oder dreifach, durch gleiche oder verschiedene Reste aus der Reihe (C₁-C₄)-Alkyl, (C₁-C₄)-Alkoxy, Halogen, Nitro, Trifluormethyl, Hydroxy, Methylendioxy, Cyan, Hydroxycarbonyl, Aminocarbonyl, (C₁-C₄)-Alkoxycarbonyl, Phenyl, Phenoxy, Benzyloxy substituiert sein.

Beispiele für Pyridyl sind 2-Pyridyl, 3-Pyridyl und 4-Pyridyl.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Bevorzugt sind in zweifach substituierten Phenylresten die beiden Substituenten in der 3- und der 4-Posititon, bezogen auf die Verknüpfungsstelle, angeordnet. Entsprechendes gilt für Phenylenreste.

Der Pyridylrest ist ein Beispiel für einen Stickstoffheterocyclus, der an einem oder mehreren Kohlenstoffatomen durch (C₁-C₄)-Alkyl, Halogen, Hydroxy, (C₁-C₄)-Alkoxy, z. B. Methoxy, Phenyl-(C₁-C₄)-alkoxy, z. B. Benzyloxy, substituiert sein kann und der auch als N-Oxid vorliegen kann, beispielsweise als 2-, 3- oder 4-Pyridyl-N-oxid.

Halogen steht für Fluor, Chlor, Brom oder Jod, insbesondere für Fluor oder Chlor.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin, Ethanolamin oder Tris-(2-hydroxy-ethyl)-amin.

Verbindungen der allgemeinen Formel I, welche basische Gruppen, z. B. eine Aminogruppe, eine Amidinogruppe oder eine Guanidinogruppe enthalten, bilden mit anorganischen Säuren, wie z. B. Salzsäure, Schwefelsäure oder Phosphorsäure, und mit organischen Carbon- oder Sulfonsäuren, wie z. B. Essigsäure, Citronensäure, Benzoesäure, Maleinsäure, Fumarsäure, Weinsäure, Methansulfonsäure oder p-Toluolsulfonsäure Salze.

Die erfindungsgemäßen Verbindungen der allgemeinen Formel I können optisch aktive Kohlenstoffatome, die unabhängig voneinander R- oder S-Konfiguration haben können, enthalten und somit in Form reiner Enantiomerer oder reiner Diastereomerer oder in Form von Enantiomerengemischen oder Diastereomerengemischen vorliegen. Sowohl reine Enantiomere und Enantiomerengemische als auch Diastereomere und Diastereomerengemische sind Gegenstand der vorliegenden Erfindung. Natürliche und unnatürliche Aminosäuren können, falls chiral, in der D- oder L-Form vorliegen.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Ein bevorzugter Alkylrest, für den R¹³ steht, ist der Methylrest.

Bevorzugte Verbindungen der allgemeinen Formel I sind solche, worin R³ für gegebenenfalls substituiertes Phenyl oder Naphthyl oder für CONHR¹⁴ steht, wobei -NHR¹⁴ für den Rest der α-Aminosäure Phenylglycin oder deren (C₁-C₈)-Alkylester steht. Der für -NHR¹⁴ stehende Rest der α-Aminosäure wird dabei formal durch Abstraktion eines Wasserstoffatoms von der Aminogruppe der Aminosäure erhalten.

Darüber hinaus bevorzugt sind Verbindungen der allgemeinen Formel I, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für den Rest CONHR¹⁴ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁴ für Methyl steht, das durch Phenyl und Hydroxycarbonyl substituiert ist, oder für Methyl steht, das durch Phenyl und (C₁-C₈)-Alkoxycarbonyl, bevorzugt (C₁-C₄)-Alkoxycarbonyl, substituiert ist;
h für 1 oder 2, bevorzugt für 1, steht.

Steht -NHR¹⁴ für einen (C₁-C₈)-Alkylester einer α-Aminosäure bzw. enthält R¹⁴ einen Alkoxycarbonylrest, so ist der Methyl-, Ethyl-, Isopropyl-, Isobutyl- oder tert.-Butylester bevorzugt.

Bevorzugte Verbindungen der allgemeinen Formel I sind weiterhin solche, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für den Rest CONHR¹⁵ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
h für 1 oder 2, bevorzugt für 1, steht.

Sodann sind besonders bevorzugte Verbindungen der allgemeinen Formel I auch solche, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen Phenylrest steht; R¹⁰ für Hydroxy oder (C₁-C₈)-Alkoxy, insbesondere (C₁-C₄)-Alkoxy, steht, und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
h für 1 oder 2, bevorzugt für 1, steht.

Unter diesen besonders bevorzugten Verbindungen der allgemeinen Formel I sind ganz besonders bevorzugt diejenigen, worin gleichzeitig die Gruppe R¹-A-C(R¹³) für R¹-A-C(CH₃) steht, das heißt, R¹³ für Methyl steht;
die Gruppe R⁰N für HN steht, das heißt, R⁰ für Wasserstoff steht;
A für einen 1,4-Phenylenrest steht;
R¹ für einen Amino-imino-methyl-Rest steht;
die Gruppe CH(R³) für CH(Phenyl) steht, das heißt, R³ für Phenyl steht;
die Gruppe C(=O)-R¹⁰ für Hydroxycarbonyl, Methoxycarbonyl, Ethoxycarbonyl oder Isopropoxycarbonyl steht, das heißt, R¹⁰ für Hydroxy, Methoxy, Ethoxy oder Isopropoxy steht;
R für Wasserstoff steht;
h für 1 steht.

Darüber hinaus bevorzugt sind von diesen ganz besonders bevorzugten Verbindungen diejenigen, die an dem Chiralitätszentrum in der 4-Position des Imidazolidin-Ringes und dem für D stehenden chiralen Kohlenstoffatom jeweils eine einheitliche Konfiguration aufweisen, insbesondere an dem für D stehenden Kohlenstoffatomen die S-Konfiguration.

Auch bei allen bevorzugten Ausführungsformen umfaßt die vorliegende Erfindung natürlich, wie oben schon gesagt, die physiologisch verträglichen Salze der Verbindungen.

Verbindungen der Formel I können beispielsweise hergestellt werden durch Fragmentkondensation einer Verbindung der allgemeinen Formel II 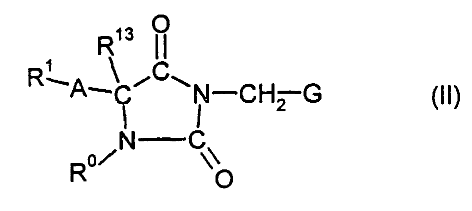 mit einer Verbindung der allgemeinen Formel III, 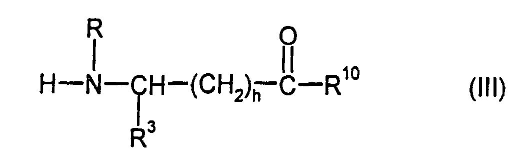 wobei A, R, R⁰, R¹, R³, R¹⁰ und R¹³ und h wie oben angegeben definiert sind und G für Hydroxycarbonyl, (C₁-C₆)-Alkoxycarbonyl, aktivierte Carbonsäurederivate, wie Säurechloride, Aktivester oder gemischte Anhydride, steht.

Zur Kondensation der Verbindungen der allgemeinen Formel II mit denen der allgemeinen Formel III verwendet man vorteilhafterweise die an sich bekannten Kupplungsmethoden der Peptidchemie (siehe z. B. Houben-Weyl, Methoden der Organischen Chemie, Band 15/1 und 15/2, Stuttgart, 1974). Dazu ist es in der Regel nötig, daß vorhandene, nicht reagierende Aminogruppen durch reversible Schutzgruppen während der Kondensation geschützt werden. Gleiches gilt für die Carboxylgruppen der Verbindungen der Formel III, die bevorzugt als (C₁-C₆)-Alkyl-, Benzyloder tert.-Butylester vorliegen. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert.-Butyltyp werden sauer gespalten, während der 9-Fluorenylmethyloxycarbonylrest durch sekundäre Amine entfernt wird.

Die Verbindungen der allgemeinen Formel I können auch wie folgt erhalten werden:

Durch Reaktion von α-Aminosäuren oder N-substituierten α-Aminosäuren oder bevorzugt deren Ester, z.B. der Methyl-, Ethyl-, tert.-Butyl- oder Benzylester, beispielsweise einer Verbindung der allgemeinen Formel IV, 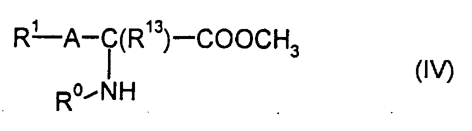 worin R⁰, R¹, R¹³ und A wie oben angegeben definiert sind, mit einem Isocyanat der allgemeinen Formel V, 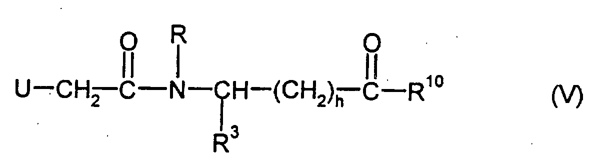 worin R, R³, R¹⁰ und h wie oben
angegeben definiert sind und U Isocyanato
oder Trichlormethylcarbonylamino bedeutet, erhält man Harnstoffderivate, beispielsweise der allgemeinen Formel VI, 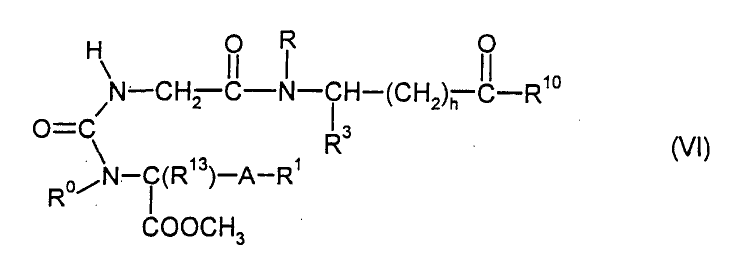 für die die oben angegebenen Definitionen gelten, die durch Erhitzen mit Säure unter Verseifung der Esterfunktionen zu Verbindungen der allgemeinen Formel I cyclisiert werden.

Während der Cyclisierung können Guanidinogruppen durch Schutzgruppen, wie NO₂ oder Mtr, blockiert werden. Ebenso können Aminogruppen in der Seitenkette in geschützter Form (Beispielsweise als Boc-oder Z-Derivat) oder noch als NO₂- oder Cyanofunktion vorliegen, die später zur Aminogruppe reduziert oder im Falle der Cyanogruppe auch in die Amidinogruppe umgewandelt werden kann.

Eine weitere Methode zur Herstellung von Verbindungen der allgemeinen Formel I ist die Umsetzung von Verbindungen der allgemeinen Formel VII, 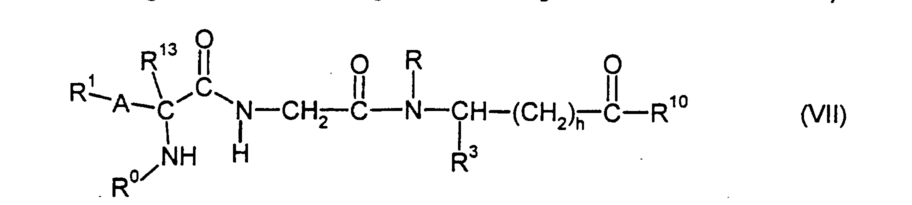 für die die
oben gegebenen Definitionen gelten, mit Phosgen oder entsprechenden Äquivalenten (analog S. Goldschmidt und M. Wick, Liebigs Ann. Chem. 575(1952), 217 - 231, und C. Tropp, Chem. Ber. 61(1928), 1431 - 1439).

Für die Guanylierung und Nitroguanylierung der Aminofunktion können folgende Reagentien verwendet werden:
1. O-Methylisoharnstoff
   (S. Weiss und H. Krommer, Chemiker-Zeitung 98 (1974) 617 - 618),
2. S-Methylisothioharnstoff
   R.F. Borne, M.L. Forrester und I.W. Waters, J. Med. Chem. 20 (1977) 771 - 776),
3. Nitro-S-methylisothioharnstoff
   (L.S. Hafner und R.E. Evans, J. Org. Chem. 24 (1959) 1157),
4. Formamidinsulfonsäure
   (K. Rim, Y.-T. Lin und H.S. Mosher, Tetrahedron Lett. 29 (1988) 3183 - 3186),
5. 3,5-Dimethyl-1-pyrazolyl-formamidinium-nitrat
   (F.L. Scott, D.G. O'Donovan und J. Reilly, J. Amer. Chem. Soc. 75 (1953) 4053 - 4054),
6. N,N'-Di-tert.-butyloxycarbonyl-S-methyl-isothioharnstoff
   (R.J. Bergeron und J.S. McManis, J. Org. Chem. 52 (1987) 1700 - 1703),
7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers,
   A. Widding, P. Andrews, H.-P. Schulz und H. Thomas, Arzneim. Forsch./Drug Res. 34 (1984) 531 - 542).

Amidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließende Aminolyse, z.B. durch Behandlung mit Ammoniak in Alkoholen wie z.B. Isopropanol, Ethanol oder Methanol, hergestellt werden (G. Wagner, P. Richter und Ch. Garbe, Pharmazie 29 (1974) 12-15). Eine weitere Methode, Amidine herzustellen, ist die Anlagerung von H₂S an die Cyanogruppe, gefolgt von einer Methylierung des entstandenen Thioamids und anschließender Umsetzung mit Ammoniak (DDR-Patent Nr. 235 866).

Die Verbindungen der allgemeinen Formel I und ihre physiologisch verträglichen Salze können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z. B. in Form von Suppositorien, oder parenteral, z. B. in Form von Injektions- oder Infusionslösungen, Mikrokapseln oder Rods, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihre pharmakologisch annehmbaren Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüber hinaus lassen sich die Verbindungen beispielsweise auch mit nootrop wirksamen Substanzen, wie z. B. Piracetam, oder ZNS-aktiven Substanzen, wie Pirlindol, Sulpirid etc. kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg, Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg, Körpergewicht. Die Tagesdosis kann, insbesondere bei der Applikation größerer Mengen, in mehrere, z. B. 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines seiner pharmazeutisch akzeptablen Salze pro Dosis.

Die erfindungsgemäßen Verbindungen der allgemeinen Formel I haben die Fähigkeit, die Zell-Zell-Adhäsion zu hemmen, die auf der Interaktion von Arg-Gly-Asp-enthaltenden Proteinen, wie Fibronectin, Fibrinogen oder des von Willebrand-Faktors, mit den sogenannten Integrinen beruhen. Integrine sind Transmembran-Glykoproteine, Rezeptoren für Arg-Gly-Asp-enthaltende Proteine (E. Ruoslahti und M. D. Pierschbacher, Science 238 (1987) 491 - 497; D. R. Phillips, I. F. Charo, L. V. Parise und L. A. Fitzgerald, Blood 71 (1988) 831 -843). Außerdem hemmen sie die Bindung weiterer adhäsiver Proteine wie Vitronectin, Kollagen und Laminin an die entsprechenden Rezeptoren auf der Oberfläche verschiedener Zelltypen.

Die erfindungsgemäßen Verbindungen der allgemeinen Formel I hemmen die Thrombozytenaggregation, die Metastasierung von Karzinomzellen sowie die Osteoclastenbindung an die Knochenoberflächen.

Die Verbindungen der allgemeinen Formel I finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, dissezierendem Aneurysma; bei der Therapie venöser und mikrozirkulatorischer Gefäßerkrankungen, wie tiefer Venenthrombose, disseminierter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist bei der Krebsbehandlung gegeben, z.B. während Krebsoperationen und auch prophylaktisch bei Krebs. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen (verwendet werden gelfiltrierte Blutplättchen aus humanem Spenderblut, die mit ADP oder Thrombin aktiviert werden), sowie auf ihre in vivo-Wirkung zur Hemmung der Thrombozytenaggregation und Thrombosehemmung.

### Testmethode 1

Als funktioneller Test wird die Hemmung der Aggregation gelfiltrierter Human-Thrombozyten nach ADP- oder Thrombin-Stimulierung durch die erfindungsgemäßen Verbindungen gemessen. Angegeben ist der IC₅₀-Wert der Hemmung [Literatur: Marguerie, G. A. et al., J. Biol. Chem. 254, 5357-5363 (1979); Marguerie, G. A. et al., J. Biol. Chem 255, 154-161 (1980)].

Hierzu wurden Human-Thrombozyten aus plättchenreichem Plasma (PRP) durch Gelfiltration an Sepharose 2 B isoliert. Die erhaltene Suspension von gelfiltrierten Plättchen (GFP), die 3•10⁸ Plättchen/ml enthielt, wurde in Gegenwart von 1 mg/ml Fibrinogen entweder mit 10 µM ADP oder mit 0.1 U/ml Thrombin aktiviert und bei 37 °C bei 1 000 Umdrehungen pro Minute in einem Aggregometer (PAP 4, Biodata, Hatboro, PA, USA) gerührt. Als Maß für die Aggregation wird die maximale Zunahme der Lichtdurchlässigkeit gemessen. Die Testsubstanzen wurden bei 37 °C 2 min vor der Aktivierung mit ADP bzw. Thrombin zu den GFP gegeben. Die Hemmung der Aggregation wird als IC₅₀-Wert angegeben, d. h. als die mittlere Konzentration an Testsubstanz, die für eine 50 %ige Hemmung in GFP-Proben von 2-4 verschiedenen Spendern benötigt wird (halblogarithmische Dosis-Wirkungs-Beziehung).

Bei diesem Test wurden für die Verbindungen der nachstehenden Beispiele folgende Ergebnisse erhalten:

| Beispiel: | ADP stimuliert IC₅₀ (µM) | Thrombin stimuliert IC₅₀ (µM) |
|---|---|---|
| 1 | 0.04 | 0.05 |
| | | |
| 8 | 0.15 | 0.1 |
| | | |
| 10 | 0.15 | 0.06 |
| 13 | 0.2 | 0.2 |
| | | |
| 21 | 0.8 | 0.5 |
| 22 | 0.025 | 0.05 |
| 23 | 0.03 | 0.05 |
| 24 | 0.055 | 0.08 |
| | | |
| 26 | 0.02 | 0.04 |
| 27 | 0.025 | 0.04 |
| 28 | 0.025 | 0.04 |
| 29 | 0.05 | 0.04 |
| | | |
| 35 | 0.1 | 0.3 |
| 36 | 0.2 | 0.15 |
| 37 | 0.1 | 0.2 |
| 38 | 0.3 | 0.35 |
| 39 | 0.08 | 0.15 |
| 40 | 0.4 | 0.25 |
| 41 | 0.1 | 0.15 |
| 42 | 0.3 | 0.2 |
| 43 | 0.5 | 0.5 |
| 44 | 0.4 | 0.2 |
| 45 | 0.2 | 0.2 |
| 46 | 0.1 | 0.1 |
| 47 | 0.1 | 0.15 |
| 48 | 2.0 | 0.8 |
| 49 | 0.6 | 0.2 |
| 50 | 0.55 | 0.4 |
| 52 | 0.5 | 0.4 |
| 56 | 0.1 | 0.06 |
| 57 | 6 | 5 |
| 58 | 0.02 | 0.025 |
| 59 | 50 | 40 |
| 60 | 5 | 4 |
| 67 | 0.08 | 0.2 |
| 68 | 0.05 | 0.045 |
| 69 | 0.025 | 0.045 |
| 70 | 0.065 | 0.07 |

### Testmethode 2

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der Kᵢ-Wert der Bindungshemmung von ¹²⁵I-Fibrinogen nach Stimulierung mit ADP (10µM) [Literatur: Bennett, J. S.; Vilaire, G. J. Clin. Invest. 64, 1393-1401 (1979); Kornecki, E. et al., J. Biol. Chem. 256, 5696-5701 (1981)].

Hierzu wurden Human-Thrombozyten aus plättchenreichem Plasma (PRP) durch Gelfiltration an Sepharose 2 B isoliert. Es wurde eine Suspension von gelfiltrierten Plättchen (GFP) erhalten, die 4•10⁸ Plättchen/ml enthielt. Die Plättchen wurden in Gegenwart von 40 nmol/l ¹²⁵I-Fibrinogen, 10 µM ADP und verschiedenen Konzentrationen der Testsubstanz für 30 min bei Raumtemperatur inkubiert. Dann wurden aliquote Teile von 100 µl auf 20 %ige Sucrose gegeben und die Plättchen durch 2 minütige Zentrifugation bei 12 000 Umdrehungen pro Minute sedimentiert. Der Überstand wurde sorgfältig und vollständig dekantiert und der verbliebene Bodensatz wurde in einem Gammazähler vermessen. Durch Subtraktion der Bindung in Gegenwart eines Überschusses (10 µM) von unmarkiertem Fibrinogen von der gesamten gebundenen Radioaktivität wurde die spezifische Bindung ermittelt. Die Bindung wird in fmol ¹²⁵I-Fibrinogen/10⁸ Plättchen angegeben. Die Dissoziationskonstante Kᵢ für die Testsubstanz wurde aus den Verdrängungsexperimenten ¹²⁵I-Fibrinogen vs. (nicht-markierte) Testsubstanz durch eine Computeranalyse der Bindungsdaten bestimmt (Sigma-Plot).

Bei diesem Test wurden für die Verbindungen der nachstehenden Beispiele folgende Ergebnisse erhalten:

| Beispiel: | Kᵢ(µM), ADP stimuliert |
|---|---|
| 1 | 0.0132 |
| 56 | 0.0218 |
| 57 | 1.97 |
| 58 | 0.0092 |

### Testmethode 3

Geprüft wird die durch die erfindungsgemäßen Verbindungen bewirkte Hemmung der Bindung von Fibrinogen an seinen Rezeptor (Glykoprotein IIb/IIIa) am isolierten Rezeptor, der aus humanen Thrombozyten isoliert und in Mikrotiterplatten immobilisiert wurde. Angegeben ist der Kᵢ-Wert der Bindungshemmung von ¹²⁵I-Fibrinogen [Literatur: Fitzgerald, L.A. et al., Anal.Biochem. 151, 169-177 (1985) Pytela, R. et al., Science 231, 1559-1562 (1986); Charo, I.F. et al., J.Biol.Chem. 266, 1415-1421 (1991); Scarborough, R.M. et al., J.Biol.Chem. 266, 9359-9362 (1991)]. Bei diesem Test wurden für die Verbindungen der nachstehenden Beispiele folgende Ergebnisse erhalten:

| Beispiel: | Kᵢ(nM), ADP stimuliert |
|---|---|
| 1 | 0.172 |
| 13 | 0.748 |
| 21 | 1.9 |
| 22 | 0.15 |
| | |
| 27 | 0.107 |
| 26 | 0.117 |
| 28 | 0.078 |
| 39 | 0.948 |
| 40 | 1.99 |
| 46 | 1.23 |
| 56 | 0.486 |
| 57 | 37.3 |
| 58 | 0.172 |

### BEISPIELE

Die Produkte wurden über Massenspektren und/oder NMR-Spektren identifiziert.

### Beispiel 1:

### ((R,S)-4-(4-(Amino-imino-methyl)-phenyl)-4-methyl-2,5-dioxoimidazolidin-1-yl)-acetyl-L-aspartyl-L-phenylglycin

### 1a. (R,S)-4-(4-Cyanophenyl)-4-methyl-2,5-dioxoimidazolidin

20 g (138 mMol) p-Acetylbenzonitril, 115,6 g Ammoniumcarbonat (1.21 Mol) and 11,6 g Kaliumcyanid (178 mMol) werden in 600 ml einer Mischung aus 50% Ethanol und 50% Wasser gelöst. Das Gemisch wird 5 Stunden bei 55°C gerührt und über Nacht bei Raumtemperatur stehen gelassen. Die Lösung wird mit 6 N HCl auf pH = 6,3 eingestellt und anschließend zwei Stunden bei Raumtemperatur gerührt. Der Niederschlag wird abgesaugt, mit Wasser gewaschen und über Phosphorpentoxid im Hochvakuum getrocknet. Ausbeute: 22,33 g (75%).

### 1b. ((R,S)-4-(4-Cyanophenyl)-4-methyl-2,5-dioxoimidazolidin-1-yl)-essigsäure-methylester

1,068 g Natrium (46,47 mMol) werden unter Stickstoff in 110 ml abs. Methanol gelöst. Die klare Lösung wird mit 10 g (R,S)-4-(4-Cyanophenyl)-4-methyl-2,5-dioxoimidazolidin (46,47 mMol) versetzt und das Gemisch wird 2 h unter Rückfluß gekocht. Man gibt 7,75 g (46,68 mMol) Kaliumiodid zu und tropft innerhalb einer Stunde eine Lösung von 4,53 ml Chloressigsäure-methylester (51,3 mMol) in 5 ml Methanol zu. Es wird 6 Stunden zum Sieden erhitzt, über Nacht bei Raumtemperatur stehen gelassen und eingeengt. Der ölige Rückstand wird über Kieselgel mit Methylenchlorid/Essigsäureethylester (9:1) chromatographiert.
Ausbeute: 8,81 g (66%).

### 1c. ((R,S)-4-(4-(Ethoxy-imino-methyl)-phenyl-4-methyl-2,5-dioxoimidazolidin-1-yl)-essigsäure-methylester-hydrochlorid

Eine Suspension von 4 g ((R,S)-4-(4-Cyanophenyl)-4-methyl-2,5-dioxoimidazolidin-1-yl)-essigsäure-methylester (13,92 mMol) in 60 ml abs. Ethanol wird auf 0°C abgekühlt. Trockenes HCl-Gas wird in die Suspension eingeleitet, wobei die Temperatur stets unter 10°C gehalten wird, bis im IR-Spektrum die Nitril-Bande nicht mehr vorliegt. Die ethanolische Lösung wird mit 200 ml Diethylether versetzt und über Nacht bei 4°C stehen gelassen. Der Niederschlag wird abgesaugt und im Hochvakuum getrocknet.
Ausbeute: 3,96 g (77 %).

### 1d. ((R,S)-4-(4-(Amino-imino-methyl)-phenyl)-4-methyl-2,5-dioxoimidazolidin-1-yl)-essigsäure-methylesterhydrochlorid

3,96 g ((R,S)-4-(4-(Ethoxy-imino-methyl)-phenyl)-4-methyl-2,5-dioxoimidazolidin-1-yl)-essigsäure-methylesterhydrochlorid (10,7 mMol) werden in 40 ml Isopropanol suspendiert und mit 11,9 ml einer 2 N Lösung von Ammoniak in Isopropanol versetzt. Das Reaktionsgemisch wird 2 Stunden bei 50°C gerührt. Der Ansatz wird abgekühlt und dann mit 200 ml Diethylether versetzt. Der Niederschlag wird abgesaugt und im Hochvakuum getrocknet.
Ausbeute: 3,27 g (89 %).

### 1e. ((R,S)-4-(4-(Amino-imino-methyl)-phenyl)-4-methyl-2,5-dioxoimidazolidin-1-yl)-essigsäure-hydrochlorid

3,27 g ((R,S)-4-(4-(Amino-imino-methyl)-phenyl)-4-methyl-2,5-dioxoimidazolidin-1-yl)-essigsäure-methylester-hydrochlorid (9,6 mMol) werden in 50 ml konzentrierter Salzsäure gelöst. Die Lösung wird 6 Stunden zum Sieden erhitzt und dann eingeengt.
Ausbeute: 2,73 g (87 %).

### 1f. ((R,S)-4-(4-(Amino-imino-methyl)-phenyl)-4-methyl-2,5-dioxoimidazolidin-1-yl)-acetyl-L-aspartyl-L-phenylglycin-di-tert.-butylester-hydrochlorid

Zu einer Lösung von 1 g ((R,S)-4-(4-(Amino-imino-methyl)-phenyl)-4-methyl-2,5-dioxoimidazolidin-1-yl)-essigsäurehydrochlorid (3,06 mMol), 1,27 g H-Asp(OBu^{t})-Phg-OBu^{t}-Hydrochlorid (3,06 mMol) und 413 mg HOBt in 10 ml Dimethylformamid gibt man bei 0°C 673 mg DCC (3,06 mMol). Man läßt eine Stunde bei 0°C und 4 Stunden bei Raumtemperatur rühren. Anschließend läßt man den Ansatz übers Wochenende im Kühlraum stehen, saugt den Niederschlag ab und engt das Filtrat ein. Zur Reinigung wird die Substanz über Kieselgel mit Methylenchlorid/Methanol/Eisessig/Wasser (8,5:1,5:0,15:0,15) chromatographiert.
Ausbeute: 920 mg Öl (enthält noch Essigsäure).

### 1g. ((R,S)-4-(4-(Amino-imino-methyl)-phenyl)-4-methyl-2,5-dioxoimidazolidin-1-yl)-acetyl-L-aspartyl-L-phenyl glycin

920 mg ((R,S)-4-(4-(Amino-imino-methyl)-phenyl)-4-methyl-2,5-dioxoimidazolidin-1-yl)-acetyl-L-aspartyl-L-phenylglycin-di-tert.-butylester-hydrochlorid werden in einer Mischung aus 5,4 ml Trifluoressigsäure, 0,6 ml Wasser und 0,6 ml Dimercaptoethan gelöst. Man läßt eine Stunde bei Raumtemperatur stehen und engt im Wasserstrahlvakuum ein. Zur Reinigung wird die Substanz an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 390 mg
[α]_{D} = + 1,3° (c = 1, in Methanol, 25°C).

### Beispiel 2:

### ((R,S)-4-(4-(Amino-imino-methyl)-phenyl)-3,4-dimethyl-2,5-dioxoimidazolidin-1-yl)-acetyl-L-aspartyl-L-phenylglycin

### 2a. ((R,S)-4-(4-Cyanophenyl)-3,4-dimethyl-2,5-dioxoimidazolid in-1-yl)-essigsäure-methylester

3 g ((R,S)-4-(4-Cyanophenyl)-4-methyl-2,5-dioxoimidazolidin-1-yl)-essigsäure-methylester (10,4 mMol) werden unter Argon in 15 ml wasserfreiem Dimethylformamid gelöst. Im Argon-Gegenstrom werden 275,5 mg einer Natriumhydrid-Dispersion in Mineralöl (11,4 mMol) zugegeben. Das Reaktionsgemisch wird 15 Minuten bei Raumtemperatur gerührt. Anschließend versetzt man mit 721 µl Methyliodid (11,4 mMol). Es wird 4 Stunden bei Raumtemperatur gerührt und dann über Nacht bei Raumtemperatur stehen gelassen. Die Lösung wird konzentriert. Zur Reinigung wird die Substanz über Kieselgel mit Methylenchlorid/Essigsäureethylester (9,5:0,5) chromatographiert. Die Fraktionen mit der reinen Substanz werden eingeengt.
Ausbeute: 2,14 g Öl (68 %).

### 2b. ((R,S)-4-(4-(Ethoxy-imino-methyl)-phenyl)-3,4-dimethyl-2,5-dioxoimidazolidin-1-yl)-essigsäure-methylester-hydrochlorid

Eine Lösung von 2,56 g ((R,S)-4-(4-Cyanophenyl)-3,4-dimethyl-2,5-dioxoimidazolidin-1-yl)-essigsäure-methylester (8,5 mMol) in 40 ml abs. Ethanol wird auf 0°C abgekühlt. Trockenes HCl-Gas wird in die Lösung eingeleitet wobei die Temperatur stets unter 10°C gehalten wird, bis im IR-Spektrum die Nitril-Bande nicht mehr vorliegt. Die ethanolische Lösung wird auf 20 ml eingeengt und mit 200 ml Diethylether versetzt. Die Suspension wird eingeengt und im Hochvakuum getrocknet.
Ausbeute: 2,27 g (76 %).

### 2c. ((R,S)-4-(4-(Amino-imino-methyl)-phenyl)-3,4-dimethyl-2,5-dioxoimidazolidin-1-yl)-essigsäure-methylester-hydrochlorid

2,26 g ((R,S)-4-(4-(Ethoxy-imino-methyl)-phenyl)-3,4-dimethyl-2,5-dioxo-imidazolidin-1-yl)-essigsäure-methylester-hydrochlorid (6,4 mMol) werden in 25 ml Isopropanol suspendiert und mit 7,2 ml einer 2 N Lösung von Ammoniak in Isopropanol versetzt. Das Reaktionsgemisch wird 2,5 Stunden bei 50°C gerührt. Der Ansatz wird abgekühlt und dann mit 200 ml Diethylether versetzt. Der Niederschlag wird abgesaugt und im Hochvakuum getrocknet.
Ausbeute: 1,03 g (45 %).

### 2d. ((R,S)-4-(4-(Amino-imino-methyl)-phenyl)-3,4-dimethyl-2,5-dioxoimidazolidin-1-yl)-essigsäure-hydrochlorid

1 g ((R,S)-4-(4-(Amino-imino-methyl)-phenyl)-3,4-dimethyl-2,5-dioxoimidazolidin-1-yl)-essigsäure-methylester-hydrochlorid (3,14 mMol) werden in 20 ml konzentrierter Salzsäure gelöst. Die Lösung wird 6 Stunden zum Sieden erhitzt und dann eingeengt.
Ausbeute: 770 mg (81 %).

### 2e. ((R,S)-4-(4-(Amino-imino-methyl)-phenyl)-3,4-dimethvl-2,5-dioxoimidazolidin-1-yl)-acetyl-L-aspartyl-Lphenylglycin-di-tert.-butylester-hydrochlorid

Zu einer Lösung von 340 mg ((R,S)-4-(4-(Amino-imino-methyl)-phenyl)-3,4-dimethyl-2,5-dioxoimidazolidin-1-yl)-essigsäure-hydrochlorid (1 mMol), 415 mg H-Asp(OBu^{t})-Phg-OBut-Hydrochlorid (1 mMol) und 135 mg HOBt in 7 ml Dimethylformamid gibt man bei 0°C 220 mg DCC (1 mMol). Man setzt 0,13 ml N-Ethylmorpholin, zu bis ein pH von 5,0 erreicht ist, und läßt eine Stunde bei 0°C und 2 Stunden bei Raumtemperatur rühren. Anschließend läßt man den Ansatz übers Wochenende im Kühlraum stehen, saugt den Niederschlag ab und engt das Filtrat ein. Zur Reinigung wird die Substanz an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 377 mg (57%).

### 2f. ((R,S)-4-(4-(Amino-imino-methyl)-phenyl)-3,4-dimethyl-2,5-dioxoimidazolidin-1-yl)-acetyl-L-aspartyl-Lphenylglycin

370 mg ((R,S)-4-(4-(Amino-imino-methyl)-phenyl)-3,4-dimethyl-2,5-dioxo-imidazolidin-1-yl)-acetyl-L-aspartyl-Lphenylglycin-di-tert.-butylester-hydrochlorid (0,53 mMol) werden in einer Mischung aus 3,6 ml Trifluoressigsäure, 0,4 ml Wasser und 0,4 ml Dimercaptoethan gelöst. Man läßt eine Stunde bei Raumtemperatur stehen und engt im Wasserstrahlvakuum ein. Zur Reinigung wird die Substanz an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 210 mg eines weißen Feststoffes (72 %).
[α]_{D} = -2,8° (c = 1, in Methanol, 23°C).

### Beispiel 3:

### ((R,S)-4-(4-(Amino-imino-methyl)-phenyl)-4-methyl-2,5-dioxoimidazolidin-1-yl)-acetyl-L-aspartyl-L-phenylglycin dimethylester-hydrochlorid

Zu einer Lösung von 1,47 g ((R,S)-4-(4-(Amino-iminomethyl)-phenyl)-4-methyl-2,5-dioxoimidazolidin-1-yl)-essigsäure-hydrochlorid (4,4 mMol), 1,45 g H-Asp(OMe)-Phg-OMe-Hydrochlorid (4,4 mMol) und 600 mg HOBt in 15 ml Dimethylformamid gibt man bei 0°C 977 mg DCC (5,66 mMol). Man läßt eine Stunde bei 0°C und 8 Stunden bei Raumtemperatur rühren. Man saugt den Niederschlag ab und engt das Filtrat ein. Zur Reinigung wird die Substanz über Kieselgel mit Methylenchlorid/Methanol/Eisessig/Wasser (8:2:0, 15:0,15) und anschließend in Methylenchlorid/Methanol/ Eisessig (30:10:0,5) chromatographiert. Die Fraktionen mit der reinen Substanz werden eingeengt. Der Rückstand wird in Wasser gelöst und gefriergetrocknet.
Ausbeute: 437 mg eines weißen Feststoff (16 %).

### Beispiel 4:

### ((R,S)-4-(4-(Amino-imino-methyl)-phenyl)-4-methyl-2,5-dioxoimidazolidin-1-yl)-acetyl-L-aspartyl-L-phenylglycindiisopropylester-hydrochlorid

### 4a. (N-Benzyloxycarbonyl)-L-phenylglycin-isopropylester

20 g Z-Phg-OH (70 mMol) werden in einer Mischung aus 26 ml Isopropanol und 26 ml Pyridin gelöst. Man setzt eine Lösung von 31,5 ml 50% Propanphosphonsäureanhydrid in Essigsäureethylester und 350 mg DMAP zu und rührt für 24 Stunden bei Raumtemperatur. Der Ansatz wird anschließend im Vakuum eingeengt und der Rückstand wird zwischen Essigsäureethylester und Wasser verteilt. Die organische Phase wird mit einer Kaliumhydrogensulfat-Lösung (100 g Kaliumsulfat und 50 g Kaliumhydrogensulfat gelöst in 1 Liter Wasser), einer Natriumhydrogencarbonat-Lösung und mit Wasser ausgeschüttelt. Die organische Phase wird über Natriumsulfat getrocknet und eingeengt.
Ausbeute: 16,74 g Öl (73 %)

### 4b. L-Phenylglycin-isopropylester-hydrochlorid

16,74 g (N-Benzyloxycarbonyl)-L-phenylglycin-isopropylester (51 mMol) werden in Methanol gelöst und an der Autobürette unter Zugabe von 2 N methanolischer HCl bei einem pH von 4,6 über Pd/Aktivkohle katalytisch hydriert. Der Katalysator wird über Kieselgur abgesaugt und das Filtrat eingeengt. Der Rückstand wird mit Diethylether verrieben.
Ausbeute: 9,21 g eines weißen Feststoffes (79 %).

### 4c. L-Asparaginsäure-C_{β}-isopropylester-hydrochlorid

1000 ml Isopropanol werden auf -10°C abgekühlt und langsam mit 31 ml (0,16 Mol) Thionylchlorid versetzt. In die Lösung werden 40 g L-Asparaginsäure (0,3 Mol) eingetragen. Das Gemisch wird 6 Stunden bei 40°C gerührt. Anschließend läßt man den Ansatz übers Wochenende bei Raumtemperatur stehen. Die Lösung wird auf ein Volumen von 250 ml eingeengt und mit 500 ml Diethylether versetzt. Der Niederschlag wird abgesaugt. Das Filtrat wird weiter eingeengt und weiteres Rohprodukt wird durch Zugabe von Diethylether ausgefällt. Zur Reinigung werden 20 g des Rohproduktes auf einer Säule mit 1 kg saurem Aluminiumoxid gereinigt.
Ausbeute: 8,55 g

### 4d. (N-Benzyloxycarbonyl)-L-asparaginsäure-C_{β}-isopropylester-cyclohexylaminsalz

8,55 g L-Asparaginsäure-C_{β}-isopropylester-hydrochlorid (48,8 mMol) werden in einem Gemisch aus 110 ml Wasser und 110 ml Dioxan gelöst und mit 4,1 g (48,8 mMol) Natriumhydrogencarbonat versetzt. Man setzt 13,4 g N-(Benzyloxycarbonyl-oxy)-succinimid (53,8 mMol) zu und rührt für 1 Stunde bei Raumtemperatur. Durch Zugabe von 10 g Natriumhydrogencarbonat wird der pH auf einen Wert von 8 eingestellt. Der Ansatz wird 5 Stunden bei Raumtemperatur gerührt und dann eingeengt. Der Rückstand wird zwischen Essigsäureethylester und 2 N HCl verteilt. Die organische Phase wird mit Wasser ausgeschüttelt, über Natriumsulfat getrocknet und eingeengt. Das erhaltenene Öl (12,35 g) löst man in 300 ml Diethylether. Zu der Lösung wird Cyclohexylamin zugetropft, bis ein pH von 8,0 erreicht wird. Der Niederschlag wird abgesaugt und mit Diethylether gewaschen.
Ausbeute: 12,84 g (64%).

### 4e. (N-Benzyloxycarbonyl)-L-asparaginsäure-C_{β}-isopropylester

12,84 g (N-Benzyloxycarbonyl)-L-asparaginsäure-C_{β}-isopropylester-cyclohexylamin-salz (31,4 mMol) werden in 250 ml Essigsäureethylester suspendiert. Die Suspension wird mit 15,7 ml einer 2 N Schwefelsäure (31,4 mMol) und Wasser ausgeschüttelt, bis eine klare Lösung entsteht. Die organische Phase wird mit Kaliumhydrogensulfat-Lösung (100 g Kaliumsulfat und 50 g Kaliumhydrogensulfat gelöst in 1 Liter Wasser) gewaschen, über Natriumsulfat getrocknet und eingeengt.
Ausbeute: 8,22 g Öl (85%).

### 4f. (N-Benzyloxycarbonyl)-L-asparaginsäure-C_{β}-isopropylester-L-phenylglycin-isopropylester

Zu einer Lösung von 8 g Z-L-Asp(OiPr)-OH (25,86 mMol), 5,94 g H-Phg-OiPr (25,86 mMol) und 3,49 g HOBt in 100 ml Dimethylformamid gibt man bei 0°C 3,36 ml N-Ethylmorpholin und 5,69 g DCC (25,86 mMol). Man läßt eine Stunde bei 0°C und 4 Stunden bei Raumtemperatur rühren. Anschließend läßt man den Ansatz über Nacht stehen, saugt den Niederschlag ab und engt das Filtrat ein. Der Rückstand wird in Essigsäureethylester gelöst und die organische Phase wird mit einer Kaliumhydrogensulfat-Lösung (100 g Kaliumsulfat und 50 g Kaliumhydrogensulfat gelöst in 1 Liter Wasser), mit Natriumhydrogencarbonat-Lösung und mit Wasser ausgeschüttelt. Man trocknet über wasserfreiem Natriumsulfat und engt ein. Der ölige Rückstand wird über Kieselgel mit n-Heptan/Essigsäureethylester (7:3) chromatographiert.
Ausbeute: 10,28 g (82 %).

### 4g. L-Asparaginsäure-C_{β}-isopropylester-L-phenylglycinisopropylester-hydrochlorid

10,28 g (N-Benzyloxycarbonyl)-L-asparaginsäure-C_{β}-isopropylester-L-phenylglycin-isopropylester (21,2 mMol) werden in 250 ml Methanol gelöst und an der Autobürette unter Zugabe von 2 N methanolischer HCl bei einem pH von 4,6 über Pd/Aktivkohle katalytisch hydriert. Der Katalysator wird über Kieselgur abgesaugt und das Filtrat eingeengt. Der Rückstand wurde in Wasser aufgenommen und gefriergetrocknet.
Ausbeute: 6,56 g eines weißen Feststoffes (80 %).

### 4h. ((R,S)-4-(4-(Amino-imino-methyl)-phenyl)-4-methyl-2,5-dioxoimidazolidin-1-yl)-acetyl-L-aspartyl-L-phenylglycin-diisopropylester-hydrochlorid

Zu einer Lösung von 2 g ((R,S)-4-(4-(Amino-imino-methyl)-phenyl)-4-methyl-2,5-dioxoimidazolidin-1-yl)-essigsäurehydrochlorid (6,12 mMol), 2,37 g H-Asp(OiPr)-Phg-OiPr-Hydrochlorid (6,12 mMol) und 826,3 mg HOBt in 15 ml Dimethylformamid gibt man bei 0°C 1,35 g DCC (6,12 mMol).

Man läßt eine Stunde bei 0°C und 5 Stunden bei Raumtemperatur rühren. Anschließend läßt man den Ansatz über Nacht im Kühlraum stehen, saugt den Niederschlag ab und engt das Filtrat ein. Zur Reinigung wird die Substanz über Kieselgel mit Methylenchlorid/Methanol/Eisessig/Wasser (8,5:1,5:0,15:0,15) chromatographiert. Die Fraktionen mit der reinen Substanz werden eingeengt. Der Rückstand wird in Wasser gelöst und gefriergetrocknet.
Ausbeute: 1,03 g eines weißen Feststoff (27%).
[α]_{D} = - 9,3° (c = 1, in Methanol, 24°C).

### Beispiel 8 :

### ((R,S)-4-(4-(Aminomethyl)-phenyl)-4-methyl-2,5-dioxoimidazolidin-1-yl)-acetyl-L-aspartyl-L-phenylglycin

### 8a. ((R,S)-4-(4-(Aminomethyl)-phenyl)-4-methyl-2,5-dioxo- imidazolidin-1-yl)-essigsäure-methylester-acetat

1 g ((R,S)-4-(4-Cyanophenyl)-4-methyl-2,5-dioxoimidazolidin-1-yl)-essigsäure-methylester (3,48 mMol; siehe Beispiel 1) werden in einer Mischung aus 8 ml Ethanol und 2 ml 50 %-iger Essigsäure gelöst. Die Lösung wird mit 200 mg 10% Pd/C versetzt und 2 Stunden bei Raumtemperatur im Schüttelautoklaven bei einem Druck von 3 bar hydriert. Der Katalysator wird über Kieselgur abgesaugt und das Filtrat wird eingeengt. Der ölige Rückstand wird über Kieselgel mit Methylenchlorid/Methanol (8:2) chromatographiert.
Ausbeute: 800 mg (79 %).

### 8b. ((R,S)-4-(4-(Aminomethyl)-phenyl)-4-methyl-2,5-dioxoimidazolidin-1-yl)-essigsäure-hydrochlorid.

750 mg ((R,S)-4-(4-(Aminomethyl)-phenyl)-4-methyl-2,5-dioxoimidazolidin-1-yl)-essigsäure-methylester-acetat (2,57 mMol) werden in 15 ml konzentrierter HCl gelöst. Die Lösung wird 6 Stunden zum Sieden erhitzt und dann eingeengt. Der Rückstand wird in Wasser aufgenommen und gefriergetrocknet.
Ausbeute: 700 mg ( 87 %).

### 8c. ((R,S)-4-(4-(tert.-Butoxycarbonylaminomethyl)-phenyl)-4-methyl-2,5-dioxoimidazolidin-1-yl)-essigsäure

300 mg ((R,S)-4-(4-(Aminomethyl)-phenyl)-4-methyl-2,5-dioxoimidazolidin-1-yl)-essigsäure-hydrochlorid (0,96 mMol) werden in einer Mischung aus 2 ml Dioxan und 1 ml Wasser gelöst. Die Lösung wird mit 1 N NaOH (ca. 1 ml) auf einen pH von 8,0 eingestellt und anschließend auf 0°C abgekühlt. Es werden unter Rühren 230 mg Di-tert-butyl-dicarbonat (1,05 mMol) zugegeben. Man läßt das Reaktionsgemisch auf Raumtemperatur erwärmen und rührt weitere 3 Stunden. Der pH wird dabei durch kontinuierliche Zugabe von 1 N NaOH (ca. 1,2 ml) auf einem Wert von 8,0 gehalten. Das Reaktionsgemisch wird im Vakuum aufkonzentriert. Der Rückstand wird unter Kühlung (0°C) mit einer Kaliumhydrogensulfat-Lösung (100 g Kaliumsulfat und 50 g Kaliumhydrogensulfat gelöst in 1 Liter Wasser) auf einen pH von 2,0 eingestellt. Die wäßrige Phase wird dreimal mit Essigsäureethylester extrahiert. Man schüttelt die vereinigten organischen Phasen mit Wasser aus und trocknet über wasserfreiem Natriumsulfat. Die organische Phase wird eingeengt. Der Rückstand wird in wenig Wasser aufgenommen und gefriergetrocknet.
Ausbeute: 340 mg (94%).

### 8d. ((R,S)-4-(4-(tert.-Butoxycarbonylaminomethyl)-phenyl)-4-methyl-2,5-dioxoimidazolidin-1-yl)-acetyl-Laspartyl-L-phenylglycin-di-tert.-butylester

Zu einer Lösung von 300 mg ((R,S)-4-(4-(tert.-Butoxycarbonylaminomethyl)-phenyl)-4-methyl-2,5-dioxoimidazolidin-1-yl)-essigsäure (0,8 mMol), 332 mg H-Asp(OBu^{t})-Phg-OBu^{t}-Hydrochlorid (0,8 mMol) und 108 mg HOBt in 3 ml Dimethylformamid gibt man bei 0°C 104 µl N-Ethylmorpholin und 176 mg DCC (0,9 mMol). Man läßt eine Stunde bei 0°C und 4,5 Stunden bei Raumtemperatur rühren. Anschließend läßt man den Ansatz über Nacht im Kühlraum stehen, saugt den Niederschlag ab und engt das Filtrat ein. Zur Reinigung wird die Substanz über Kieselgel mit Methylenchlorid/Methanol (20:1) chromatographiert.
Ausbeute: 320 mg Öl (54%).

### 8e. ((R,S)-4-(4-(Aminomethyl)-phenyl)-4-methyl-2,5-dioxoimidazolidin-1-yl)-acetyl-L-asparthyl-L-phenylglycin

270 mg ((R,S)-4-(4-(tert.-Butoxycarbonylaminomethyl)-phenyl)-4-methyl-2,5-dioxoimidazolidin-1-yl)-acetyl-Laspartyl-L-phenylglycin-di-tert.-butylester (0,51 mMol) werden in einer Mischung aus 1,8 ml Trifluoressigsäure, 0,2 ml Wasser und 0,2 ml Dimercaptoethan gelöst. Nach einer Stunde bei Raumtemperatur wird im Wasserstrahlvakuum eingeengt. Zur Reinigung wird die Substanz an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 160 mg (59%)
[α]_{D} = + 1,7° (c = 1, in Methanol, 23°C).

### Beispiel 10:

### ((R,S)-4-(4-Guanidino-phenyl)-4-methyl-2,5-dioxoimidazolidin-1-yl)-acetyl-L-aspartyl-L-phenylglycin

### 10a. ((R,S)-4-(4-Benzyloxycarbonyl-guanidino-phenyl)-4-methyl-2,5-dioxoimidazolidin-1-yl)-acetyl-L-aspartyl-Lphenylglycin-di-tert.-butylester

300 mg (0,625 mMol) ((R,S)-4-(4-Benzyloxycarbonyl-guanidino-phenyl)-4-methyl-2,5-dioxoimidazolidin-1-yl)-essigsäure (Bsp. 11e) werden in 50 ml Dimethylformamid gelöst und bei 0°C mit 145 mg (0,7 mMol) DCC und 85 mg (0,625 mMol) HOBt versetzt. Man rührt 1 Stunde nach, gibt 260 mg (0,625 mMol) H-Asp(OBu^{t})-Phg-OBu^{t}-hydrochlorid und 86,4 mg (0,75 mMol) N-Ethylmorpholin zu. Man rührt 4 Stunden bei Raumtemperatur, engt ein, löst in Essigester, saugt ab und wäscht die organische Phase mit Natriumhydrogencarbonatlösung und Kaliumhydrogensulfatlösung, trocknet und engt ein. Der Rückstand wird mit Ether verrührt und abgesaugt.
Ausbeute. 370 mg (74%)

### 10b. ((R,S)-4-(4-Guanidino-phenyl)-4-methyl-2,5-dioxoimidazolidin-1-yl)-acetyl-L-aspartyl-L-phenylglycin

370 mg (0,46 mMol) ((R,S)-4-(4-Benzyloxycarbonyl-guanidino-phenyl)-4-methyl-2,5-dioxoimidazolidin-1-yl)-acetyl-L-aspartyl-L-phenylglycin-di-tert.-butylester werden mit 3.7 ml 90%iger Trifluoressigsäure 1 Stunde bei Raumtemperatur gerührt und die Lösung anschließend im Hochvakuum eingeengt. Der Rückstand wird in 50 ml Methanol gelöst, mit 50 mg 10%-Pd auf Kohle versetzt und bei Raumtemperatur hydriert. Nach vollständiger Reaktion wird vom Katalysator abfiltriert, eingeengt und der Rückstand zur Reinigung an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute. 123 mg (48%)
Schmelzpunkt: 180°C

### Beispiel 12:

### (R,S)-3-(((R,S)-4-(4-(Amino-imino-methyl)-phenyl)-4-methyl-2,5-dioxoimidazolidin-1-yl)-acetylamino)-3-phenylpropionsäure-methylester-hydrochlorid

Zu einer Lösung von 653 mg ((R,S)-4-(4-(Amino-iminomethyl)-phenyl)-4-methyl-2,5-dioxoimidazolidin-1-yl)-essigsäure-hydrochlorid (2 mMol), 358 mg (R,S)-3-Amino-3-phenyl-propionsäure-methylester (2 mMol) und 270 mg HOBt in 10 ml Dimethylformamid gibt man bei 0°C 440 mg DCC (2 mMol). Man läßt eine Stunde bei 0°C und 3 Stunden bei Raumtemperatur rühren. Anschließend läßt man den Ansatz über Nacht stehen, saugt den Niederschlag ab und engt das Filtrat ein. Zur Reinigung wird die Substanz (1,8 g) an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 597 mg (61 %).

### Beispiel 13:

### (R,S)-3-(((R,S)-4-(4-(Amino-imino-methyl)-phenyl)-4-methyl-2,5-dioxoimidazolidin-1-yl)-acetylamino)-3-phenylpropionsäure-hydrochlorid

580 mg (R,S)-3-(((R,S)-4-(4-(Amino-imino-methyl)-phenyl)-4-methyl-2,5-dioxoimidazolidin-1-yl)-acetylamino)-3-phenyl-propionsäure-methylester-hydrochlorid (1,19 mMol) werden in 55 ml konzentrierter Salzsäure gelöst und 5,5 Stunden bei Raumtemperatur stehen gelassen. Die Lösung wird eingeengt. Zur Reinigung wird die Substanz (540 mg) an Sephadex LH20 mit einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 477 mg (85 %).
[a]_{D} = + 2,5° (c = 1, in Wasser, 23°C).

### Beispiel 14:

### ((R,S)-4-(4-Guanidino-phenyl)-4-methyl-2,5-dioxoimidazolidin-1-yl)-acetyl-L-aspartyl-L-phenylglycin-dimethylester-hydrochlorid

Die Verbindungen der Beispiele 21 und 22 sind Diastereomere.

### Beispiel 21

### ((S oder R)-4-(4-(Amino-imino-methyl)-phenyl)-4-methyl-2,5-dioxoimidazolidin-1-yl)-acetyl-L-aspartyl-L-phenylglycin

### Diastereomer I

Das Diastereomeren-Gemisch von ((R,S)-4-(4-(Amino-iminomethyl)-phenyl)-4-methyl-2,5-dioxoimidazolidin-1-yl)-acetyl-L-aspartyl-L-phenylglycin (Beispiel 1) wird durch Chromatographie an einer LiChroprep-RP-18-Reversed-Phase-Säule (10µm) unter Verwendung eines Wasser/Acetonitril-Gemisches (880 ml Wasser; 120 ml Acetonitril; 1 ml Trifluoressigsäure) als Laufmittel getrennt. Fraktionen, die den zuerst von der Säule eluierenden Peak enthalten, werden eingeengt. Der Rückstand wird in wenig Wasser aufgenommen und gefriergetrocknet.
[a]_{D} = -14° (c =1, in Wasser, 30°C).
FAB-MS: 539 (M+H)⁺

### Beispiel 22:

### ((R oder S)-4-(4-(Amino-imino-methyl)-phenyl)-4-methyl-2,5-dioxoimidazolidin-1-yl)-acetyl-L-aspartyl-L-phenyl glycin

### Diastereomer II

Analog Beispiel 21 wird das Diastereomer II aus dem Diastereomeren-Gemisch von ((R,S)-4-(4-(Amino-imino-methyl)-phenyl)-4-methyl-2,5-dioxoimidazolidin-1-yl)-acetyl-Laspartyl-L-phenylglycin durch Chromatographie an einer LiChroprep-RP-18-Reversed-Phase-Säule (10µm) isoliert. Dazu werden die Fraktionen eingeengt, die den als zweiten von der Säule eluierenden Peak enthalten. Der Rückstand wird in wenig Wasser aufgenommen und gefriergetrocknet.
[a]_{D} = +20° (c =1, in Wasser, 30°C).
FAB-MS: 539 (M+H)⁺

### Beispiel 23:

### ((R,S)-4-(4-(Amino-imino-methyl)-phenyl)-4-methyl-2,5-dioxoimidazolidin-1-yl)-acetyl-L-aspartyl-L-phenylglycinmethylester

FAB-MS: 553 (M+H)⁺

### Beispiel 24:

### ((R,S)-4-(4-(Amino-imino-methyl)-phenyl)-3-ethyl-4-methyl-2,5-dioxoimidazolidin-1-yl)-acetyl-L-aspartyl-Lphenylglycin

FAB-MS: 466 (M+H)⁺

### Beispiel 26:

### ((R,S)-4-(4-(Amino-imino-methyl)-phenyl)-4-cyclopropyl-2,5-dioxoimidazolidin-1-yl)-acetyl-L-aspartyl-L-phenylglycin

### 26a. 4-Cyanphenyl-cyclopropyl-methanon

22,5 g 4-Bromphenyl-cyclopropyl-methanon (100 mMol) und 10,3 g CuCN (100 mMol) werden 15 ml DMF gelöst und 4 Stunden unter Rühren am Rückfluß erhitzt. Man läßt auf 70°C abkühlen und gießt die Suspension in eine Lösung aus 40 g Eisen-(III)-chlorid, 10 ml konz. HCl und 60 ml Wasser. Es wird 20 Minuten bei 70°C gerührt. Man extrahiert dreimal mit je 90 ml Toluol. Die vereinigten organischen Phasen werden mit 250 ml 2N Salzsäure sowie mit 250 ml 2N Natronlauge gewaschen und eingeengt. Der feste Rückstand wird mit Petrolether verrieben und abgesaugt.
Ausbeute 14,57 g (85%)
FAB-MS: 172 (M+H)⁺

### 26b. ((R,S)-4-(4-(Amino-imino-methyl)-phenyl)-4-cyclopropyl-2,5-dioxoimidazolidin-1-yl)-acetyl-L-aspartyl-Lphenylglycin

Die Synthese erfolgt ausgehend von 4-Cyanphenyl-cyclopropyl-methanon analog zu Beispiel 1.
FAB-MS: 565 (M+H)⁺

### Beispiel 27:

### ((R,S)-4-(4-(Amino-imino-methyl)-phenyl)-4-ethyl-2,5-dioxoimidazolidin-1-yl)-acetyl-L-aspartyl-L-phenylglycin

Die Synthese erfolgt ausgehend von 1-(4-Bromphenyl)-1-propanon analog zu Beispiel 26.
FAB-MS: 553 (M+H)⁺

### Beispiel 28:

### ((R,S)-4-(4-(Amino-imino-methyl)-phenyl)-4-benzyl-2,5-dioxoimidazolidin-1-yl)-acetyl-L-aspartyl-L-phenylglycin

Die Synthese erfolgt ausgehend von 2-Phenyl-1-(4-Bromphenyl)-1-ethanon analog zu Beispiel 26.
FAB-MS: 615 (M+H)⁺

### Beispiel 29:

### ((R,S)-4-(4-(Amino-imino-methyl)-phenyl)-4-tert.-butyl-2,5-dioxoimidazolidin-1-yl)-acetyl-L-aspartyl-L-phenylglycin

### 29a. 4-Bromphenyl-tert.-butyl-methanon

21 g frisch gepulvertes, wasserfreies Kaliumhydroxid (375 mMol) werden mit 50 ml wasserfreiem Toluol überschichtet. Man setzt 20 mg 18-Krone-6 (0,75 mMol) sowie 9,95 g 4-Bromacetophenon (50 mMol) zu. Es wird auf 70°C erwärmt. Man versetzt die Reaktionslösung langsam mit 24,94 ml Iodmethan (395 mMol) und rührt 3,5 Stunden bei 70°C. Die organische Phase wird mit Wasser extrahiert. Die wäßrige Phase wird zweimal mit Diethylether extrahiert. Die organischen Phasen werden vereinigt, über Natriumsulfat getrocknet und eingeengt. Das Rohprodukt (11,46 g) wird nochmals nach obigem Verfahren alkyliert, da die Umsetzung noch nicht vollständig abgelaufen war. Das erhaltene Produkt (10,86 g) wird durch Hochvakuumdestillation mittels einer Silbermantelkolonne gereinigt.
Ausbeute: 3,9 g (32 %)
FAB-MS: 242 (M+H)⁺

### 29b. ((R,S)-4-(4-(Amino-imino-methyl)-phenyl)-4-tert.-butyl-2,5-dioxoimidazolidin-1-yl)-acetyl-L-aspartyl-L-phenylglycin

Die Synthese erfolgt ausgehend von 4-Bromphenyl-tert.butyl-methanon analog zu Beispiel 26.
FAB-MS: 581 (M+H)⁺

### Beispiel 35:

### (R,S)-3-(((R,S)-4-(4-(Amino-imino-methyl)-phenyl)-4-methyl-2,5-dioxoimidazolidin-1-yl)-acetylamino)-3-(3-hydroxy-4-methoxy-phenyl)-propionsäure

FAB-MS: 484 (M+H)⁺

### Beispiel 36:

### (R,S)-3-(((R,S)-4-(4-(Amino-imino-methyl)-phenyl)-4-methyl-2,5-dioxoimidazolidin-1-yl)-acetylamino)-3-(4-hydroxy-3-methoxy-phenyl)-propionsäure

FAB-MS: 484 (M+H)⁺

### Beispiel 37:

### (R,S)-3-(((R,S)-4-(4-(Amino-imino-methyl)-phenyl)-4-methyl-2,5-dioxoimidazolidin-1-yl)-acetylamino)-3-(4-ethoxy-phenyl)-propionsäure

FAB-MS: 482 (M+H)⁺

### Beispiel 38:

### (R,S)-3-(((R,S)-4-(4-(Amino-imino-methyl)-phenyl)-4-methyl-2,5-dioxoimidazolidin-1-yl)-acetylamino)-3-(1-naphthyl)-propionsäure

FAB-MS: 488 (M+H)⁺

### Beispiel 39:

### (R,S)-3-(((R,S)-4-(4-(Amino-imino-methyl)-phenyl)-4-methyl-2,5-dioxoimidazolidin-1-yl)-acetylamino)-3-(3-nitrophenyl)-propionsäure

FAB-MS: 483 (M+H)⁺

### Beispiel 40

### (R,S)-3-(((R,S)-4-(4-(Amino-imino-methyl)-phenyl)-4-methyl-2,5-dioxoimidazolidin-1-yl)-acetylamino)-3-(4-hydroxycarbonyl-phenyl)-propionsäure

FAB-MS: 482 (M+H)⁺

### Beispiel 41:

### (R,S)-3-(((R,S)-4-(4-(Amino-imino-methyl)-phenyl)-4-methyl-2,5-dioxoimidazolidin-1-yl)-acetylamino)-3-(3-benzyloxy-phenyl)-propionsäure

FAB-MS: 544 (M+H)⁺

### Beispiel 42:

### (R,S)-3-(((R,S)-4-(4-(Amino-imino-methyl)-phenyl)-4-methyl-2,5-dioxoimidazolidin-1-yl)-acetylamino)-3-(3-hy droxycarbonyl-phenyl)-propionsäure

FAB-MS: 482 (M+H)⁺

### Beispiel 43:

### (R,S)-3-(((R,S)-4-(4-(Amino-imino-methyl)-phenyl)-4-methyl-2,5-dioxoimidazolidin-1-yl)-acetylamino)-3-(3-phen oxy-phenyl)-propionsäure

FAB-MS: 530 (M+H)⁺

### Beispiel 44:

### (R,S)-3-(((R,S)-4-(4-(Amino-imino-methyl)-phenyl)-4-methyl-2,5-dioxoimidazolidin-1-yl)-acetylamino)-3-(3,4,5-trimethoxy-phenyl)-propionsäure

FAB-MS: 528 (M+H)⁺

### Beispiel 45:

### (R,S)-3-(((R,S)-4-(4-(Amino-imino-methyl)-phenyl)-4-methyl-2,5-dioxoimidazolidin-1-yl)-acetylamino)-3-(4-hydroxy-phenyl)-propionsäure

FAB-MS: 454 (M+H)⁺

### Beispiel 46:

### (R,S)-3-(((R,S)-4-(4-(Amino-imino-methyl)-phenyl)-4-methyl-2,5-dioxoimidazolidin-1-yl)-acetylamino)-3-(4-phenyl-phenyl)-propionsäure

FAB-MS: 514 (M+H)⁺

### Beispiel 47:

### (R,S)-3-(((R,S)-4-(4-(Amino-imino-methyl)-phenyl)-4-methyl -2,5-dioxoimidazolidin-1-yl)-acetylamino)-3-(3-pyridyl)-propionsäure

FAB-MS: 439 (M+H)⁺

### Beispiel 48:

### (R,S)-3-(((R,S)-4-(4-(Amino-imino-methyl)-phenyl)-4-methyl-2,5-dioxoimidazolidin-1-yl)-acetylamino)-buttersäure

FAB-MS: 376 (M+H)⁺

### Beispiel 49:

### (R,S)-3-(((R,S)-4-(4-(Amino-imino-methyl)-phenyl)-4-methyl-2,5-dioxoimidazolidin-1-yl)-acetylamino)-3-cyclohexyl-propionsäure

FAB-MS: 444 (M+H)⁺

### Beispiel 50:

### (R,S)-3-(((R,S)-4-(4-(Amino-imino-methyl)-phenyl)-3,4-dimethyl-2,5-dioxoimidazolidin-1-yl)-acetylamino)-3- phenyl-propionsäure

FAB-MS: 452 (M+H)⁺

### Beispiel 51:

### (R,S)-3-(((R,S)-4-(4-(Amino-imino-methyl)-phenyl)-3,4-dimethyl-2,5-dioxoimidazolidin-1-yl)-acetylamino)-3-phenyl-propionsäure-ethylester-hydrochlorid

FAB-MS: 480 (M+H)⁺

### Beispiel 52:

### (R,S)-3-(((R,S)-4-(4-(Amino-imino-methyl)-phenyl)-3-ethyl-4-methyl-2,5-dioxoimidazolidin-1-yl)-acetylamino)-3-phenyl-propionsäure

FAB-MS: 466 (M+H)⁺

### Beispiel 53:

### (R,S)-3-(((R,S)-4-(4-(Amino-imino-methyl)-phenyl)-3-ethyl-4-methyl-2,5-dioxoimidazolidin-1-yl)-acetylamino)-3-phenyl-propionsäure-ethylester-hydrochlorid

FAB-MS: 494 (M+H)⁺

### Beispiel 54:

### (R,S)-3-(((R,S)-4-(4-(Amino-imino-methyl)-phenyl)-4-methyl-2,5-dioxoimidazolidin-1-yl)-acetylamino)-3-phenylpropionsäure-ethylester-hydrochlorid

FAB-MS: 466 (M+H)⁺

### Beispiel 55:

### (S)-3-(((R oder S)-4-(4-(Amino-imino-methyl)-phenyl)-4-methyl-2,5-dioxoimidazolidin-1-yl)-acetylamino)-3- phenyl-propionsäure-ethylester-hydrochlorid

Die Verbindung ist abgeleitet vom Diastereomer II des Beispiels 58.

340 mg (S)-3-(((R oder S)-4-(4-(Amino-imino-methyl)-phenyl)-4-methyl-2,5-dioxoimidazolidin-1-yl)-acetylamino)-3-phenyl-propionsäure (0,78 mMol) (Beispiel 58) werden in 60 ml 2N ethanolischer HCl-Lösung gelöst und 2 Stunden bei Raumtemperatur stehen gelassen. Man engt ein und löst den Rückstand in Wasser. Die Lösung wird filtriert und gefriergetrocknet.
Ausbeute: 375 mg eines weißen Feststoffs (96 %).
[α]_{D} = -55,5° (c = 1, in Wasser, 21°C).
FAB-MS: 466 (M+H)⁺

### Beispiel 56:

### (S)-3-(((R,S)-4-(4-(Amino-imino-methyl)-phenyl)-4-methyl-2,5-dioxoimidazolidin-1-yl)-acetylamino)-3-phenyl propionsäure-hydrochlorid

12,37 g (S)-3-(((R,S)-4-(4-(Amino-imino-methyl)-phenyl)-4-methyl-2,5-dioxoimidazolidin-1-yl)-acetylamino)-3-phenyl-propionsäure-ethylester-hydrochlorid (26,6 mMol) (Beispiel 71) werden in 200 ml konzentrierter Salzsäure gelöst und 7,5 Stunden bei Raumtemperatur stehen gelassen. Die Lösung wird eingeengt. Der Rückstand wird mit 200 ml konzentrierter Salzsäure versetzt, 7,5 Stunden bei Raumtemperatur stehen gelassen und die Lösung eingeengt. Es werden 11,6 g Rohprodukt erhalten.

Zur Reinigung wird ein Teil der Substanz (255 mg) an Sephadex LH20 in einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 232 mg.
FAB-MS: 438 (M+H)⁺

### Beispiel 57:

### Diastereomer I:

### (S)-3-(((S oder R)-4-(4-(Amino-imino-methyl)-phenyl)-4-methyl-2,5-dioxoimidazolidin-1-yl)-acetylamino)-3-phenyl propionsäure

Das Diastereomeren-Gemisch von (S)-3-(((R,S)-4-(4-(Aminoimino-methyl)-phenyl)-4-methyl-2,5-dioxoimidazolidin-1-yl)-acetylamino)-3-phenyl-propionsäure-hydrochlorid (Beispiel 56) wird durch Chromatographie an einer LiChroprep-RP-18-Reversed-Phase-Säule (10 µm) unter Verwendung eines Wasser/Acetonitril-Gemisches (920 ml Wasser; 80 ml Acetonitril; 1 g Ammoniumacetat) als Laufmittel getrennt. Dazu werden auf eine Säule mit einem Füllvolumen von 450 ml jeweils 500 mg des Diastereomeren-Gemisches aufgetragen. Fraktionen, die den zuerst von der Säule eluierenden Peak enthalten, werden eingeengt. Durch dreimaliges Gefriertrocknen wird das Ammoniumacetat entfernt.

Ausbeute pro Säulenlauf: 245 mg (49 %)
[a]_{D}=-110.4° (c=1, in Wasser, 30°C)
FAB-MS: 438 (M+H)⁺

### Beispiel 58:

### Diastereomer II:

### (S)-3-(((R oder S)-4-(4-(Amino-imino-methyl)-phenyl)-4-methyl-2,5-dioxoimidazolidin-1-yl)-acetylamino)-3-phenyl propionsäure

Analog Beispiel 57 wird das Diastereomer II aus dem Diastereomeren-Gemisch von (S)-3-(((R,S)-4-(4-(Amino-iminomethyl)-phenyl)-4-methyl-2,5-dioxoimidazolidin-1-yl)-acetylamino)-3-phenyl-propionsäure-hydrochlorid (Beispiel 56) durch Chromatographie an einer LiChroprep-RP-18-Reversed-Phase-Säule (10 µm) isoliert. Dazu werden die Fraktionen eingeengt, die den als zweiten von der Säule eluierenden Peak enthalten. Durch dreimaliges Gefriergetrocknen wird das Ammoniumacetat entfernt.
Ausbeute pro Säulenlauf: 200 mg (40 %).
[a]_{D}=-62.8° (c=1, in Wasser, 30°C)
FAB-MS: 438 (M+H)⁺

### Beispiel 59:

### Diastereomer III:

### (R)-3-(((S oder R)-4-(4-(Amino-imino-methyl)-phenyl)-4-methyl-2,5-dioxoimidazolidin-1-yl)-acetylamino)-3-phenylpropionsäure

145 mg des Diastereomeren-Gemisches von (R)-3-(((R,S)-4-(4-(Amino-imino-methyl)-phenyl)-4-methyl-2,5-dioxoimidazolidin-1-yl)-acetylamino)-3-phenyl-propionsäure-hydrochlorid (Beispiel 61) werden durch Chromatographie an einer LiChroprep-RP-18-Reversed-Phase-Säule (10 µm) analog Beispiel 57 getrennt. Fraktionen, die den zuerst von der Säule eluierenden Peak enthalten, werden eingeengt. Durch dreimaliges Gefriertrocknen wird das Ammoniumacetat entfernt.
Ausbeute: 60 mg (41 %).
[a]_{D}=+92.7° (c=1, in Wasser, 30°C)
FAB-MS: 438 (M+H)⁺

### Beispiel 60:

### Diastereomer IV:

### (R)-3-(((R oder S)-4-(4-(Amino-imino-methyl)-phenyl)-4-methyl-2,5-dioxoimidazolidin-1-yl)-acetylamino)-3-phenylpropionsäure

Analog Beispiel 59 wird das Diastereomer IV aus dem Diastereomeren-Gemisch von (R)-3-(((R,S)-4-(4-(Amino-iminomethyl)-phenyl)-4-methyl-2,5-dioxoimidazolidin-1-yl)-acetylamino)-3-phenyl-propionsäure-hydrochlorid (Beispiel 61) durch Chromatographie an einer LiChroprep-RP-18-Reversed-Phase-Säule (10 µm) isoliert. Dazu werden die Fraktionen eingeengt, die den als zweiten von der Säule eluierenden Peak enthalten. Durch dreimaliges Gefriertrocknen wird das Ammoniumacetat entfernt.
Ausbeute: 63 mg (43 %).
[a]_{D}=+51.4° (c=1, in Wasser, 30°C)
FAB-MS: 438 (M+H)⁺

### Beispiel 61:

### (R)-3-(((R,S)-4-(4-(Amino-imino-methyl)-phenyl)-4-methyl-2,5-dioxoimidazolidin-1-yl)-acetylamino)-3-phenyl-propionsäure-hydrochlorid.

Die Substanz wird analog zu den Beispielen 71 und 56 dargestellt. Dabei geht man bei der Synthese von (S)-Phenylglycin aus.
FAB-MS: 438 (M+H)⁺

### Beispiel 62:

Die Verbindung ist abgeleitet vom Diastereomer I des Beispiels 57.

### (S)-3-(((S oder R)-4-(4-(Amino-imino-methyl)-phenyl)-4-methyl-2,5-dioxoimidazolidin-1-yl)-acetylamino)-3-phenylpropionsäure-ethylester-hydrochlorid

FAB-MS: 466 (M+H)⁺

### Beispiel 63:

Die Verbindung ist abgeleitet vom Diastereomer II des Beispiels 58.

### (S)-3-(((R oder S)-4-(4-(Amino-imino-methyl)-phenyl)-4-methyl-2,5-dioxoimidazolidin-1-yl)-acetylamino)-3-phenylpropionsäure-methylester-hydrochlorid

FAB-MS: 452 (M+H)⁺

### Beispiel 64:

Die Verbindung ist abgeleitet vom Diastereomer II des Beispiels 58.

### (S)-3-(((R oder S)-4-(4-(Amino-imino-methyl)-phenyl)-4-methyl-2,5-dioxoimidazolidin-1-yl)-acetylamino)-3-phenylpropionsäure-isopropylester-hydrochlorid

FAB-MS: 480 (M+H)⁺

### Beispiel 65:

### (R,S)-3-(((R,S)-4-(4-(Amino-imino-methyl)-phenyl)-4-methyl-2,5-dioxoimidazolidin-1-yl)-acetyl-(N-methyl-amino))-3-(3-pyridyl)-propionsäure

FAB-MS: 453 (M+H)⁺

### Beispiel 66:

### (R,S)-3-(((R,S)-4-(4-(Amino-imino-methyl)-phenyl)-4-methyl-2,5-dioxoimidazolidin-1-yl)-acetyl-(N-methylamino))-3-phenyl-propionsäure

FAB-MS: 452 (M+H)⁺

### Beispiel 67:

### (R,S)-3-(((R,S)-4-(4-(Amino-imino-methyl)-phenyl)-4-methyl-2,5-dioxoimidazolidin-1-yl)-acetylamino)-3-(3,4-methylendioxy-phenyl)-propionsäure

FAB-MS: 482 (M+H)⁺

### Beispiel 68:

### (2-((R,S)-4-(4-(Amino-imino-methyl)-phenyl)-4-methyl-2,5-dioxoimidazolidin-1-yl)-acetyl)-L-aspartyl-1-adamantylamid

### 68a. (N-Benzyloxycarbonyl)-L-Asparaginsäure-C_{β}-tert.-butylester-1-adamantyl-amid.

Zu einer Suspension von 4,2 g Z-L-Asp(OBu^{t})-OH (13 mMol), 1,97 g 1-Aminoadamantan (13 mMol) und 1,76 g HOBt (13 mMol) in 140 ml Dimethylformamid gibt man bei 0°C 1,69 ml N-Ethylmorpholin (13 mMol) und 2,86 g DCC (13 mMol). Man läßt eine Stunde bei 0°C und 3 Stunden bei Raumtemperatur rühren. Anschließend läßt man den Ansatz über Nacht stehen, saugt den Niederschlag ab und engt das Filtrat ein. Der Rückstand wird in Natriumhydrogencarbonat-Lösung aufgenommen und die wäßrige Phase wird mit Essigsäureethylester ausgeschüttelt. Die organische Phase wird mit einer Kaliumhydrogensulfat-Lösung (100 g Kaliumsulfat und 50 g Kaliumhydrogensulfat gelöst in 1 Liter Wasser), mit Natriumhydrogencarbonat-Lösung und mit Wasser ausgeschüttelt. Man trocknet über wasserfreiem Natriumsulfat und engt ein.
Ausbeute: 6,21 g (Rohprodukt).

### 68b. L-Asparaginsäure-C_{β}-tert.-butylester-1-adamantylamid-hydrochlorid.

6,21 g (N-Benzyloxycarbonyl)-L-Asparaginsäure-C_{β}-tert.butylester-1-adamantyl-amid (Rohprodukt) werden in 50 ml Methanol gelöst und an der Autobürette unter Zugabe von 2N methanolischer HCl bei einem pH von 4.6 über Pd/Aktivkohle katalytisch hydriert. Der Katalysator wird über Kieselgur abgesaugt und das Filtrat eingeengt. Der Rückstand wird mit Diethylether verrieben, abgesaugt und getrocknet.
Ausbeute: 4 g (85 % bezogen auf eingesetztes
Z-L-Asp(OBu^{t})-OH); FAB-MS (M + H)⁺ = 323.

### 68c. (2-((R,S)-4-(4-(Amino-imino-methyl)-phenyl)-4-methyl-2,5-dioxoimidazolidin-1-yl)-acetyl)-L-aspartyl-C_{β}-tert.-butylester-1-adamantyl-amid-hydrochlorid.

Zu einer Suspension von 654 mg 2-((R,S)-4-(4-(Aminoimino-methyl)phenyl)-4-methyl-2,5-dioxoimidazolidin-1-yl)-essigsäure-hydrochlorid (2 mMol; siehe Beispiel 1), 718 mg L-Asparaginsäure-C_{β}-tert.-butylester-1-adamantylamid-hydrochlorid (2 mMol) und 270 mg HOBt (2 mMol) in 20 ml Dimethylformamid gibt man bei 0°C 0,26 ml N-Ethylmorpholin (2 mMol) und 440 mg DCC (2 mMol). Man läßt eine Stunde bei 0°C und 3 Stunden bei Raumtemperatur rühren. Anschließend läßt man den Ansatz über Nacht bei Raumtemperatur stehen, saugt den Niederschlag ab und engt das Filtrat ein. Der Rückstand wird in Natriumhydrogencarbonat-Lösung aufgenommen und die wäßrige Phase wird mit Pentanol ausgeschüttelt. Die organische Phase wird mit einer Kaliumhydrogensulfat-Lösung und mit Wasser ausgeschüttelt. Man trocknet über wasserfreiem Natriumsulfat und engt ein. Der Rückstand wird mit Diethylether verrieben, abgesaugt und getrocknet.
Ausbeute: 1,35 g (Rohprodukt).

### 68d. (2-((R,S)-4-(4-(Amino-imino-methyl)-phenyl)-4-methyl-2,5-dioxoimidazolidin-1-yl)-acetyl)-L-aspartyl-1-adamantyl-amid.

1,35 g (2-((R,S)-4-(4-(Amino-imino-methyl)-phenyl)-4-methyl-2,5-dioxoimidazolidin-1-yl)-acetyl)-L-aspartyl-C_{β}-tert.-butylester-1-adamantyl-amid-hydrochlorid werden in einer Mischung von 12,15 ml Trifluoroessigsäure, 1,35 ml Wasser und 1,35 ml Dimercaptoethan gelöst. Nach einer Stunde bei Raumtemperatur wird im Wasserstrahlvakuum eingeengt. Der Rückstand wird mit Diethylether verrieben, abgesaugt und getrocknet. Zur Reinigung wird die Substanz an Sephadex LH20 in einer Mischung aus Eisessig, n-Butanol und Wasser chromatographiert. Die Fraktionen mit der reinen Substanz werden eingeengt. Der Rückstand wird in Wasser unter Zusatz von etwas Essigsäure gelöst und gefriergetrocknet.
Ausbeute: 1,02 g; FAB-MS (M + H)⁺ = 539

### Beispiel 69

### (2-((R,S)-4-(4-(Amino-imino-methyl)-phenyl)-4-methyl-2,5-dioxoimidazolidi-1-yl)-acetyl)-L-aspartyl-2-adamantyl-amid

### 69a. (N-Benzyloxycarbonyl)-L-Asparaginsäure-C_{β}-tert.- butylester-2-adamantyl-amid

Zu einer Suspension von 4,2 g Z-L-Asp(OBu^{t})-OH (13 mMol), 2,44 g 2-Aminoadamantan-hydrochlorid (13 mMol) und 1,76 g HOBt (13 mMol) in 40 ml Dimethylformamid gibt man bei 0°C 1,69 ml N-Ethylmorpholn (13 mMol) und 2,86 g DCC (13 mMol). Man läßt eine Stunde bei 0°C und 3 Stunden bei Raumtemperatur rühren. Anschließend läßt man den Ansatz über Nacht stehen, saugt den Niederschlag ab und engt das Filtrat ein. Der Rückstand wird in Natriumhydrogencarbonat-Lösung aufgenommen und die wäßrige Phase wird mit Essigsäureethylester ausgeschüttelt. Die organische Phase wird mit einer Kaliumhydrogensulfat-Lösung, mit Natriumhydrogencarbonat-Lösung und mit Wasser ausgeschüttelt. Man trocknet über wasserfreiem Natriumsulfat und engt ein.
Ausbeute: 6,32 g (Rohprodukt).

### 69b. L-Asparaginsäune-C_{β}-tert.-butylester-2-adamantyl-amid-hydrochlorid.

6,32 g (N-Benzyloxycarbonyl)-L-Asparaginsäure-C_{β}-tert.butylester-2-adamantyl-amid (Rohprodukt) werden in 50 ml Methanol gelöst und an der Autobürette unter Zugabe von 2N methanolischer HCl bei einem pH von 4.6 über Pd/Aktivkohle katalytisch hydriert. Der Katalysator wird über Kieselgur abgesaugt und das Filtrat eingeengt. Der Rückstand wird in Diethylether gelöst und eingeengt. Es wird ein amorpher Feststoff erhalten.
Ausbeute: 4 g (85 % bezogen auf eingesetztes
Z-L-Asp(OBu^{t})-OH); FAB-MS (M + H)⁺ = 323

### 69c. (2-((R,S)-4-(4-(Amino-imino-methyl)-phenyl)-4-methyl-2,5-dioxoimidazolidin-1-yl)-acetyl)-L-aspartyl-C_{β}-tert.-butylester-2-adamantyl-amid-hydrochlorid

Zu einer Suspension von 654 mg 2-((R,S)-4-(4-(Aminoimino-methyl)phenyl)-4-methyl-2,5-dioxoimidazolidin1-yl)-essigsäurehydrochlorid (2 mMol; siehe Beispiel 1), 718 mg L-Asparaginsäure-C_{β}-tert.-butylester-2-adamantylamid-hydrochlorid (2mMol) und 270 mg HOBt (2mMol) in 20 ml Dimethylformamid gibt man bei 0°C 0,26 ml N-Ethylmorpholin (2 mMol) und 440 mg DCC (2 mMol). Man läßt eine Stunde bei 0°C und 2 Stunden bei Raumtemperatur rühren. Anschließend läßt man den Ansatz über Nacht bei Raumtemperatur stehen, saugt den Niederschlag ab und engt das Filtrat ein. Der Rückstand wird in Natriumhydrogencarbonat-Lösung aufgenommen und die wäßrige Phase wird mit Pentanol ausgeschüttelt. Die organische Phase wird mit einer Kaliumhydrogensulfat-Lösung und mit Wasser ausgeschüttelt. Man trocknet über wasserfreiem Natriumsulfat und engt ein. Der Rückstand wird mit Diethylether verrieben, abgesaugt und getrocknet.
Ausbeute: 1,27 g (Rohprodukt).

### 69d. (1-((R,S)-4-(4-(Amino-imino-methyl)-phenyl)-4-methyl-2,5-dioxoimidazolidin-1-yl)-acetyl)-L-aspartyl-2-adamantyl-amid

1,27 g (2-((R,S)-4-(4-(Amino-imino-methyl)-phenyl)-4-methyl-2,5-dioxoimidazolidin-1-yl)-acetyl)-L-aspartyl-C_{β}-tert.-butylester-2-adamantyl-amid-hydrochlorid werden in einer Mischung von 11,43 ml Trifluoroessigsäure, 1,27 ml Wasser und 1,27 ml Dimercaptoethan gelöst. Nach einer Stunde bei Raumtemperatur wird im Wasserstrahlvakuum eingeengt. Der Rückstand wird mit Diethylether verrieben, abgesaugt und getrocknet. Zur Reinigung wird die Substanz an Sephadex LH20 in einer Mischung aus Eisessig, n-Butanol und Wasser chromatographiert. Die Fraktionen mit der reinen Substanz werden eingeengt. Der Rückstand wird in Wasser unter Zusatz von etwas Essigsäure gelöst und gefriergetrocknet.
Ausbeute: 615,8 mg; FAB-MS (M + H)⁺= 539

### Beispiel 70

### (2-((R,S)-4-(4-(Amino-imino-methyl)-phenyl)-4-methyl-2,5-dioxoimidazolidin-1-yl)-acetyl)-L-aspartyl-(1-adamantylmethyl)-amid.

### 70a. (N-Benzyloxycarbonyl)-L-Asparaginsäure-C_{β}-tert.-butylester-(1-adamantylmethyl)-amid

Zu einer Suspension von 3,91 g Z-L-Asp(OBu^{t})-OH (12,1 mMol), 2 g 1-Aminomethyl-adamantan (12,1 mMol) und 1,63 g HOBt (12,1 mMol) in 60 ml Dimethylformamid gibt man bei 0°C 2,66 g DCC (12,1 mMol). Man läßt eine Stunde bei 0°C und 2 Stunden bei Raumtemperatur rühen. Anschließend läßt man den Ansatz über Nacht stehen, saugt den Niederschlag ab und engt das Filtrat ein. Der Rückstand wird in Natriumhydrogencarbonat-Lösung aufgenommen und die wäßrige Phase wird mit Essigsäureethylester ausgeschüttelt. Die organische Phase wird mit einer Kaliumhydrogensulfat-Lösung, mit Natriumbicarbonat-Lösung und mit Wasser ausgeschüttelt. Man trocknet über wasserfreiem Natriumsulfat und engt ein.
Ausbeute: 6 g (Rohprodukt).

### 70b. L-Asparaginsäure-C_{β}-tert.-butylester-(1-adamantylmethyl)-amid-hydrochlorid

6 g (N-Benzyloxycarbonyl)-L-Asparaginsäure-C_{β}-tert.-butylester-(1-adamantylmethyl)-amid (Rohprodukt) werden in 50 ml Methanol gelöst und an der Autobürette unter Zugabe von 2N methanolischer HCl bei einem pH von 4.6 über Pd/Aktivkohle katalytisch hydriert. Der Katalysator wird über Kieselgur abgesaugt und das Filtrat eingeengt. Der Rückstand wird mit Diethylether verrieben, abgesaugt und getrocknet.
Ausbeute: 3,85 g (85 % bezogen auf eingesetztes
Z-L-Asp(OBu^{t})-OH); FAB-MS (M + H)⁺ = 337.

### 70c. (2-((R,S)-4-(4-(Amino-imino-methyl)-phenyl)-4-methyl-2,5-dioxoimidazolidin-1-yl)-acetyl)-L-aspartyl-C_{β}-tert.-butylester-(1-adamantylmethyl)-amid-hydrochlorid

Zu einer Suspension von 654 mg 2-((R,S)-4-(4-(Aminoimino-methyl)phenyl)-4-methyl-2,5-dioxoimidazolidin-1-yl)-essigsäure-hydrochlorid (2 mMol; siehe Beispiel 1), 746 mg L-Asparaginsäure-C_{β}-tert.-butylester-(1-adamantylmethyl)-amid-hydrochlorid (2 mMol) und 270 mg HOBt (2 mMol) in 20 ml Dimethylformamid gibt man bei 0°C 0,26 ml N-Ethylmorpholin (2 mMol) und 440 mg DCC (2 mMol). Man läßt eine Stunde bei 0°C und 3 Stunden bei Raumtemperatur rühren. Anschließend läßt man den Ansatz über Nacht bei Raumtemperatur stehen, saugt den Niederschlag ab und engt das Filtrat ein. Der Rückstand wird in Natriumhydrogencarbonat-Lösung aufgenommen und die wäßrige Phase wird mit Pentanol ausgeschüttelt. Die organische Phase wird mit einer Kaliumhydrogensulfat-Lösung und mit Wasser ausgeschüttelt. Man trocknet über wasserfreiem Natriumsulfat und engt ein. Der Rückstand wird mit Diethylether verrieben, abgesaugt und getrocknet. Ausbeute: 1,28 g (Rohprodukt).

### 70d. (2-((R,S)-4-(4-(Amino-imino-methyl)-phenyl)-4-methyl-2,5-dioxoimidazolidin-1-yl)-acetyl)-L-aspartyl-(1-adamantylmethyl)-amid

1,28 g (2-((R,S)-4-(4-(Amino-imino-methyl)-phenyl)-4-methyl-2,5-dioxoimidazolidin-1-yl)-acetyl)-L-aspartyl-C_{β}-tert.-butylester-(1-adamantylmethyl)-amidhydrochlorid werden in einer Mischung von 11,52 ml Trifluoroessigsäure, 1,28 ml Wasser und 1,28 ml Dimercaptoethan gelöst. Nach einer Stunde bei Raumtemperatur wird im Wasserstrahlvakuum eingeengt. Der Rückstand wird mit Diethylether verrieben, abgesaugt und getrocknet. Zur Reinigung wird die Substanz an Sephadex LH20 in einer Mischung aus Eisessig, n-Butanol und Wasser chromatographiert. Die Fraktionen mit der reinen Substanz werden eingeengt. Der Rückstand wird in Wasser unter Zusatz von etwas Essigsäure gelöst und gefriergetrocknet.
Ausbeute: 841,1 mg; FAB-MS (M + H)⁺ = 553.

### Beispiel 71

### (S)-3-(((R,S)-4-(4-(Amino-imino-methyl)-phenyl)-4-methyl-2,5-dioxoimidazolidin-1-yl)-acetylamino)-3-phenylpropionsäure-ethylester-hydrochlorid

### 71a. (R)-2-Amino-2-phenylethanol

20 g (920 mMol) Lithiumborhydrid werden in 420 ml absolutem Tetrahydrofuran gelöst. Man tropft unter Rühren 233,5 ml (1,84 Mol) Trimethylchlorsilan zu und setzt anschließend portionsweise innerhalb von 4 Stunden 69,5 g (0,46 Mol) (R)-Phenylglycin zu. Das Reaktionsgemisch wird über Nacht bei Raumtemperatur gerührt. Dann setzt man 690 ml Methanol zu, rührt für 2 Stunden bei Raumtemperatur und engt im Vakuum ein. Der Rückstand wird unter Rühren in 690 ml 20%iger wäßriger Kaliumhydroxid-Lösung gelöst. Die wäßrige Phase wird dreimal mit Essigsäureethylester extrahiert. Die vereinigten organischen Phasen werden mit Wasser gewaschen, über Magnesiumsulfat getrocknet und im Vakuum eingeengt.
Ausbeute: 41,2 g (65,3 %); FAB-MS (M + H)⁺ = 138.

### 71b. (R)-2-Benzyloxycarbonylamino-2-phenylethanol

40,5 g (295 mMol) (R)-2-Amino-2-phenylethanol werden in 385 ml absolutem Dimethylformamid gelöst. Man setzt unter Rühren bei 0°C 73,5 g N-(Benzyloxycarbonyl-oxy)-succinimid (295 mMol) zu und rührt für 1 Stunde bei 0°C. Das Eisbad wird entfernt und der Ansatz für 48 h bei Raumtemperatur stehen gelassen. Die Reaktionslösung wird im Vakuum eingeengt und der Rückstand anschließend in 500 ml Essigsäureethylester aufgenommen. Die organische Phase wird zweimal mit 10%iger wäßriger Citronensäure-Lösung sowie einmal mit Wasser gewaschen. Man trocknet über wasserfreiem Natriumsulfat und engt ein. Das erhaltene kristalline Rohprodukt (82,3 g) wird erneut in Essigsäureethylester gelöst. Die organische Phase wird zweimal mit 10%iger wäßriger Citronensäure-Lösung sowie einmal mit Wasser gewaschen. Anschließend kristallisiert man aus Essigsäureethylester/Petrolether um.
Ausbeute: 74,6 g (93,3 %); FAB-MS (M + H)⁺ = 272.

### 71c. ((R)-2-Benzyloxycarbonylamino-2-phenyl-ethyl)-4-methylphenylsulfonat

53,9 g (R)-2-Benzyloxycarbonylamino-2-phenylethanol (198,7 mMol) werden in einer Mischung aus 500 ml Methylenchlorid sowie 80,3 ml (993,5 mMol) Pyridin gelöst. Man setzt unter Rühren bei 0°C 45,5 g (238,4 mMol) Tosylchlorid in 240 ml Methylenchlorid zu und läßt 7 Stunden bei Raumtemperatur rühren. Es werden weitere 11,36 g Tosylchlorid (59,61 mMol) zugesetzt. Man läßt 5 Stunden bei 0°C rühren. Der Ansatz wird dann über Nacht bei Raumtemperatur stehen gelassen und im Vakum eingeengt. Der Rückstand wird in Essigsäureethylester aufgenommen. Die organische Phase wird dreimal mit 10%iger wäßriger Citronensäure-Lösung und zweimal Wasser gewaschen, über Magnesiumsulfat getrocknet und im Vakuum eingeengt. Der Rückstand wird mit Diethylether verrieben, abgesaugt, mit Diethylether gewaschen und über Phosphorpentoxid getrocknet. Ausbeute: 60,9 g (72 %). Die Mutterlauge wird eingeengt, in n-Heptan/Essigsäureethylester (6:4) aufgenommen und über Kieselgel chromatographiert. Ausbeute: 3,5 g (4,2 %).
Gesamtausbeute: 64,4 g (76,2 %); FAB-MS (M + H)⁺ = 426.

### 71d. (S)-3-Benzyloxycarbonylamino-3-phenyl-propionitril

60,5 g ((R)-2-Benzyloxycarbonylamino-2-phenyl-ethyl)-4-methylphenylsulfonat (142,2 mMol) werden in 675 ml Dimethylformamid gelöst. Man setzt 13,9 g Kaliumcyanid (213,3 mMol), 5,64 g 18-Krone-6 (21,33 mMol) und 520 mg Kaliumiodid (3,13 mMol) zu und rührt 20 Stunden bei 50°C. Die Reaktionslösung wird in 500 ml Eiswasser gegossen und anschließend 5 Stunden bei 0°C gerührt. Man saugt ab und löst den Niederschlag in Essigsäureethylester. Die organische Phase wird dreimal mit Wasser gewaschen, über Magnesiumsulfat getrocknet und im Vakuum eingeengt. Der Rückstand wird mit Diethylether verrieben, abgesaugt, mit Diethylether gewaschen und über Phosphorpentoxid getrocknet.
Ausbeute: 25,3 g (63,5 %); FAB-MS (M + H)⁺ = 281.

### 71e. (S)-3-Benzyloxycarbonylamino-3-phenyl-propionsäureethylester

15 g (S)-3-Benzyloxycarbonylamino-3-phenyl-propionitril (53,51 mMol) werden in einer Mischung aus 110 ml absolutem Ethanol und 30 ml Dioxan suspendiert. Unter Rühren und Kühlung leitet man bei 10 - 15°C HCl-Gas ein. Nach kurzer Zeit bildet sich eine klare Lösung. Man leitet weiter HCl-Gas unter Kühlung ein, bis im Dünnschichtchromatogramm kein Ausgangsmaterial mehr nachgewiesen werden kann. Es wird dann für 15 Minuten Stickstoff durch die Reaktionslösung geleitet und anschließend im Vakuum eingeengt. Der Rückstand wird bis zur bleibenden Trübung mit Wasser versetzt. Man rührt 30 Minuten bei Raumtemperatur und extrahiert anschließend die wäßrige Phase dreimal mit Essigsäureethylester. Die vereinigten organischen Phasen werden mit Wasser gewaschen, über Magnesiumsulfat getrocknet und im Vakuum eingeengt. Der Rückstand wird in Essigsäureethylester/Petrolether (1:1) aufgenommen und über Kieselgel chromatographiert.
Ausbeute: 10,55 g (60 %); FAB-MS (M + H)⁺ = 328.

### 71f. (S)-3-Amino-3-phenyl-propionsäure-ethylester-hydrochlorid

10,29 g (S)-3-Benzyloxycarbonylamino-3-phenyl-propionsäure-ethylester (31,44 mMol) werden in 125 ml Ethanol gelöst und an der Autobürette unter Zugabe von 2N ethanolischer HCl bei einem pH von 4 über Pd/Aktivkohle katalytisch hydriert. Der Katalysator wird über Kieselgur abgesaugt und das Filtrat eingeengt. Der Rückstand wird mit Diethylether verrieben, abgesaugt, mit Diethylether gewaschen und über Phosphorpentoxid getrocknet.
Ausbeute: 5,05 g (70 %); FAB-MS (M + H)⁺ = 194.

### 71g. (S)-3-(((R,S)-4-(4-(Amino-imino-methyl)-phenyl)-4-methyl-2,5-dioxoimidazolidin-1-yl)-acetylamino)-3-phenylpropionsäure-ethylester-hydrochlorid

Zu einer Lösung von 26,14 g ((R,S)-4-(4-(Amino-iminomethyl)-phenyl)-4-methyl-2,5-dioxoimidazolidin-1-yl)-essigsäure-hydrochlorid (80 mMol) (Beispiel 1), 18,37 g (S)-3-Amino-3-phenyl-propionsäure-ethylester-hydrochlorid (80 mMol) und 10,8 g HOBt in 400 ml Dimethylformamid gibt man bei 0°C 10,4 ml N-Ethylmorpholin (80 mMol) sowie 17,6 g DCC (80 mMol). Man läßt eine Stunde bei 0°C und 3 Stunden bei Raumtemperatur rühren. Anschließend läßt man den Ansatz über Nacht stehen, saugt den Niederschlag ab und engt das Filtrat ein. Zur Reinigung wird der ölige Rückstand (89 g) an Sephadex LH20 in einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 35 g (94 %); FAB-MS (M + H)⁺ = 466.

## Patentansprüche

1. 5-Ring-Heterocyclen der allgemeinen Formel I, 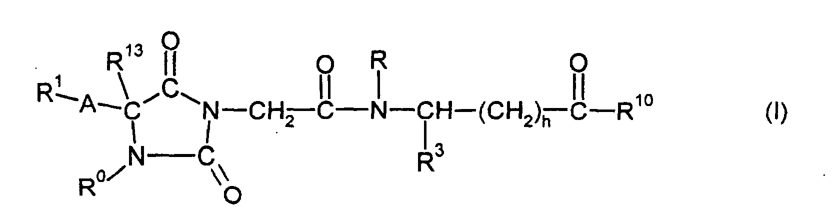 worin gleichzeitig
A für einen 1,4-Phenylenrest steht;
R und R⁰ unabhängig voneinander für Wasserstoff oder (C₁-C₄)-Alkyl stehen;
R¹ für H₂N-C(=NH), H₂N-C(=NH)-NH oder H₂N-CH₂ steht;
R³ für den Rest CONHR¹⁴ steht, für den Rest CONHR¹⁵ steht oder für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht;
R¹⁰ für Hydroxy oder (C₁-C₈)-Alkoxy steht;
R¹³ für (C₁-C₆)-Alkyl, (C₃-C₇)-Cycloalkyl oder Benzyl steht;
R¹⁴ für Methyl steht, das durch Phenyl und Hydroxycarbonyl substituiert ist, oder für Methyl steht, das durch Phenyl und (C₁-C₈)-Alkoxycarbonyl substituiert ist;
R¹⁵ für einen Adamantylrest oder einen Adamantylmethylrest steht;
h für 1 oder 2 steht;
und deren physiologisch verträgliche Salze.

2. 5-Ring-Heterocyclen der allgemeinen Formel I gemäß Anspruch 1, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für den Rest CONHR¹⁴ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁴ für Methyl steht, das durch Phenyl und Hydroxycarbonyl substituiert ist, oder für Methyl steht, das durch Phenyl und (C₁-C₈)-Alkoxycarbonyl, bevorzugt (C₁-C₄)-Alkoxycarbonyl, substituiert ist;
h für 1 oder 2, bevorzugt für 1, steht;
und deren physiologisch verträgliche Salze.

3. 5-Ring-Heterocyclen der allgemeinen Formel I gemäß Anspruch 1, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für den Rest CONHR¹⁵ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
h für 1 oder 2, bevorzugt für 1, steht;
und deren physiologisch verträgliche Salze.

4. 5-Ring-Heterocyclen der allgemeinen Formel I gemäß Anspruch 1, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für Phenyl steht;
R¹⁰ für Hydroxy oder (C₁-C₈)-Alkoxy, insbesondere (C₁-C₄)-Alkoxy, steht und R¹⁰ bevorzugt für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
h für 1 oder 2, bevorzugt für 1, steht;
und deren physiologisch verträgliche Salze.

5. 5-Ring-Heterocyclen der allgemeinen Formel I gemäß den Ansprüchen 1 und/oder 4, worin gleichzeitig
A für einen 1,4-Phenylenrest steht;
R und R⁰ unabhängig voneinander für Wasserstoff oder (C₁-C₄)-Alkyl, insbesondere Wasserstoff, Methyl oder Ethyl, stehen;
R¹ für H₂N-C(=NH), H₂N-C(=NH)-NH oder H₂N-CH₂ steht;
R³ für einen unsubstituierten Phenylrest oder einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Hydroxycarbonyl, (C₁-C₄)-Alkoxycarbonyl, Aminocarbonyl, Cyan, Phenyl, Phenoxy und Benzyloxy substituierten Phenylrest steht;
R¹⁰ für Hydroxy oder (C₁-C₈)-Alkoxy, insbesondere (C₁-C₄)-Alkoxy, steht und R¹⁰ bevorzugt für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
h für 1 oder 2, bevorzugt für 1, steht;
und deren physiologisch verträgliche Salze.

6. 5-Ring-Heterocyclen der allgemeinen Formel I gemäß einem oder mehreren der Ansprüche 1, 4 und 5, worin gleichzeitig
A für einen 1,4-Phenylenrest steht;
R für Wasserstoff steht;
R⁰ für Wasserstoff steht;
R¹ für einen Amino-imino-methyl-Rest steht;
R³ für Phenyl steht;
R¹⁰ für Hydroxy, Methoxy, Ethoxy oder Isopropoxy steht;
R¹³ für (C₁-C₆)-Alkyl, (C₃-C₇)-Cycloalkyl oder Benzyl, insbesondere Methyl, steht;
h für 1 steht,
und bevorzugt an dem Chiralitätszentrum in der 4-Position des Imidazolidin-Ringes und an dem den Rest R³ tragenden chiralen Kohlenstoffatom jeweils eine einheitliche Konfiguration vorliegt;
und deren physiologisch verträgliche Salze.

7. 5-Ring-Heterocyclen der allgemeinen Formel I gemäß einem oder mehreren der Ansprüche 1, 4, 5 und 6, worin gleichzeitig
A für einen 1,4-Phenylenrest steht;
R für Wasserstoff steht;
R⁰ für Wasserstoff steht;
R¹ für einen Amino-imino-methyl-Rest steht;
R³ für Phenyl steht;
R¹⁰ für Hydroxy, Methoxy, Ethoxy oder Isopropoxy steht;
R¹³ für Methyl steht;
h für 1 steht
und bevorzugt an dem Chiralitätszentrum in der 4-Position des Imidazolidin-Ringes und an dem den Rest R³ tragenden chiralen Kohlenstoffatom jeweils eine einheitliche Konfiguration vorliegt;
und deren physiologisch verträgliche Salze.

8. 5-Ring-Heterocyclen der allgemeinen Formel I gemäß Anspruch 7, worin R¹⁰ für Hydroxy steht, an dem Chiralitätszentrum in der 4-Position des Imidazolidinringes die S-Konfiguration vorliegt und an dem den Rest R³ tragenden chiralen Kohlenstoffatom die S-Konfiguration vorliegt, und deren physiologisch verträgliche Salze.

9. 5-Ring-Heterocyclen der allgemeinen Formel I gemäß Anspruch 7, worin R¹⁰ für Hydroxy steht, an dem Chiralitätszentrum in der 4-Position des Imidazolidinringes die R-Konfiguration vorliegt und an dem den Rest R³ tragenden chiralen Kohlenstoffatom die S-Konfiguration vorliegt, und deren physiologisch verträgliche Salze.

10. 5-Ring-Heterocyclen der allgemeinen Formel I gemäß Anspruch 7, worin R¹⁰ für Ethoxy steht, an dem Chiralitätszentrum in der 4-Position des Imidazolidinringes die S-Konfiguration vorliegt und an dem den Rest R³ tragenden chiralen Kohlenstoffatom die S-Konfiguration vorliegt, und deren physiologisch verträgliche Salze.

11. 5-Ring-Heterocyclen der allgemeinen Formel I gemäß Anspruch 7, worin R¹⁰ für Ethoxy steht, an dem Chiralitätszentrum in der 4-Position des Imidazolidinringes die R-Konfiguration vorliegt und an dem den Rest R³ tragenden chiralen Kohlenstoffatom die S-Konfiguration vorliegt, und deren physiologisch verträgliche Salze.

12. Verfahren zur Herstellung von Verbindungen der allgemeinen Formel I gemäß einem oder mehreren der Ansprüche 1 bis 11, **dadurch gekennzeichnet, daß** man eine Fragmentkondensation einer Verbindung der allgemeinen Formel II 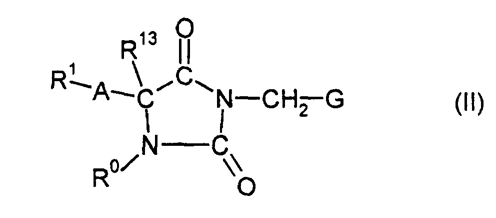 mit einer Verbindung der allgemeinen Formel III 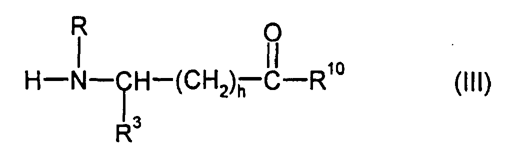 ausführt, wobei A, R, R⁰, R¹, R³, R¹⁰ und R¹³ sowie h wie in den Ansprüchen 1 bis 11 angegeben definiert sind und G für Hydroxycarbonyl, (C₁-C₆)-Alkoxycarbonyl, aktivierte Carbonsäurederivate, wie Säurechloride, Aktivester oder gemischte Anhydride steht.

13. 5-Ring-Heterocyclen der allgemeinen Formel I gemäß einem oder mehreren der Ansprüche 1 bis 11 und deren physiologisch verträgliche Salze zur Anwendung als Arzneimittel.

14. 5-Ring-Heterocyclen der allgemeinen Formel I gemäß einem oder mehreren der Ansprüche 1 bis 11 und deren physiologisch verträgliche Salze als Hemmstoffe der Thrombozytenaggregation, der Metastasierung von Karzinomzellen oder der Osteoclastenbindung an die Knochenoberfläche.

15. 5-Ring-Heterocyclen der allgemeinen Formel I gemäß einem oder mehreren der Ansprüche 1 bis 11 und deren physiologisch verträgliche Salze zur Behandlung von arteriellen, venösen oder mikrozirkulatorischen Gefäßerkrankungen, von Thrombosen oder bei Thrombosegefahr.

16. Pharmazeutisches Präparat, **dadurch gekennzeichnet, daß** es eine oder mehrere Verbindungen der allgemeinen Formel I gemäß einem oder mehreren der Ansprüche 1 bis 11 und/oder ein oder mehrere physiologisch verträgliche Salze davon als Wirkstoff zusammen mit pharmazeutisch annehmbaren Träger- und Zusatzstoffen und gegebenenfalls noch ein oder mehrere andere pharmakologische Wirkstoffe enthält.

17. Verfahren zur Herstellung eines pharmazeutischen Präparates, enthaltend eine oder mehrere Verbindungen der allgemeinen Formel I gemäß einem oder mehreren der Ansprüche 1 bis 11 und/oder ein oder mehrere physiologisch verträgliche Salze davon, **dadurch gekennzeichnet, daß** man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

## Claims

1. 5-Membered ring heterocycles of the general formula I, 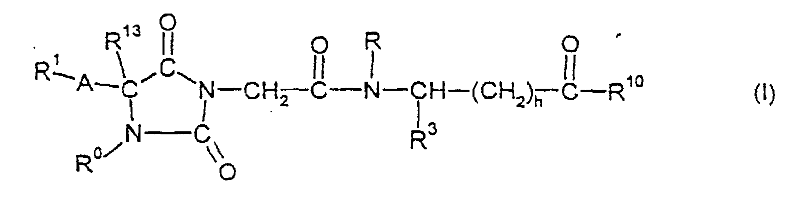 in which, at the same time,
A represents a 1,4-phenylene radical;
R and R⁰, independently of each other, represent hydrogen or (C₁-C₄)-alkyl;
R¹ represents H₂N-C(=NH), H₂N-C (=NH)-NH or H₂N-CH₂;
R³ represents the radical CONHR¹⁴, represents the radical CONHR¹⁵, or represents an unsubstituted phenyl radical or naphthyl radical, a phenyl radical or naphthyl radical which is substituted by one, two or three identical or different radicals from the group (C₁-C₄)-alkyl, (C₁-C₄)-alkoxy, hydroxyl, halogen, trifluoromethyl, nitro, methylenedioxy, hydroxycarbonyl, (C₁-C₄)-alkoxycarbonyl, aminocarbonyl, cyano, phenyl, phenoxy and benzyloxy, a pyridyl radical, a (C₁-C₄)-alkyl radical, a (C₂-C₄)-alkenyl radical, a (C₂-C₄)-alkynyl radical or a (C₅-C₆)-cycloalkyl radical;
R¹⁰ represents hydroxyl or (C₁-C₈)-alkoxy;
R¹³ represents (C₁-C₆)-alkyl, (C₃-C₇)-cycloalkyl or benzyl;
R¹⁴ represents methyl which is substituted by phenyl and hydroxycarbonyl, or represents methyl which is substituted by phenyl and (C₁-C₈)-alkoxycarbonyl;
R¹⁵ represents an adamantyl radical or an adamantyl-methyl radical;
h represents 1 or 2;
and the physiologically tolerated salts thereof.

2. 5-Membered ring heterocycles of the general formula I according to Claim 1, in which, at the same time,
A represents a 1,4-phenylene radical;
R and R⁰, independently of each other, represent hydrogen or (C₁-C₄)-alkyl, in particular hydrogen, methyl or ethyl;
R¹ represents H₂N-C(=NH), H₂N-C(=NH)-NH or H₂N-CH₂;
R³ represents the radical CONHR¹⁴;
R¹⁰ represents hydroxyl or (C₁-C₈)-alkoxy, preferably (C₁-C₄)-alkoxy;
R¹³ represents (C₁-C₆)-alkyl, (C₃-C₇)-cycloalkyl or benzyl, in particular methyl;
R¹⁴ represents methyl which is substituted by phenyl and hydroxycarbonyl, or represents methyl which is substituted by phenyl and (C₁-C₈)-alkoxycarbonyl, preferably (C₁-C₄)-alkoxycarbonyl;
h represents 1 or 2, and preferably represents 1;
and the physiologically tolerated salts thereof.

3. 5-Membered ring heterocycles of the general formula I according to Claim 1, in which, at the same time,
A represents a 1,4-phenylene radical;
R and R⁰, independently of each other, represent hydrogen or (C₁-C₄)-alkyl, in particular hydrogen, methyl or ethyl;
R¹ represents H₂N-C(=NH), H₂N-C(=NH)-NH or H₂N-CH₂;
R³ represents the radical CONHR¹⁵;
R¹⁰ represents hydroxyl or (C₁-C₈)-alkoxy, preferably (C₁-C₄)-alkoxy;
R¹³ represents (C₁-C₆)-alkyl, (C₃-C₇)-cycloalkyl or benzyl, in particular methyl;
R¹⁵ represents an adamantyl radical or an adamantyl-methyl radical;
h represents 1 or 2, and preferably represents 1;
and the physiologically tolerated salts thereof.

4. 5-Membered ring heterocycles of the general formula I according to Claim 1, in which, at the same time,
A represents a 1,4-phenylene radical;
R and R⁰, independently of each other, represent hydrogen or (C₁-C₄)-alkyl, in particular hydrogen, methyl or ethyl;
R¹ represents H₂N-C(=NH), H₂N-C(=NH)-NH or H₂N-CH₂;
R³ represents an unsubstituted phenyl radical or naphthyl radical, a phenyl radical or naphthyl radical which is substituted by one, two or three identical or different radicals from the group (C₁-C₄)-alkyl, (C₁-C₄)-alkoxy, hydroxyl, halogen, trifluoromethyl, nitro, methylenedioxy, hydroxycarbonyl, (C₁-C₄)-alkoxycarbonyl, aminocarbonyl, cyano, phenyl, phenoxy and benzyloxy, a pyridyl radical, a (C₁-C₄)-alkyl radical, a (C₂-C₄)-alkenyl radical, a (C₂-C₄)-alkynyl radical or a (C₅-C₆)-cycloalkyl radical, and, in particular R³ represents phenyl;
R¹⁰ represents hydroxyl or (C₁-C₈)-alkoxy, in particular (C₁-C₄)-alkoxy, and, preferably R¹⁰ represents a radical from the group hydroxyl, methoxy, ethoxy, propoxy and isopropoxy;
R¹³ represents (C₁-C₆)-alkyl, (C₃-C₇)-cycloalkyl or benzyl, in particular methyl;
h represents 1 or 2; and preferably represents 1;
and the physiologically tolerated salts thereof.

5. 5-Membered ring heterocycles of the general formula I according to Claims 1 and/or 4, in which, at the same time,
A represents a 1,4-phenylene radical;
R and R⁰, independently of each other, represent hydrogen or (C₁-C₄)-alkyl, in particular hydrogen, methyl or ethyl;
R¹ represents H₂N-C(=NH), H₂N-C(=NH)-NH or H₂N-CH₂;
R³ represents an unsubstituted phenyl radical or a phenyl radical which is substituted by one, two or three identical or different radicals from the group (C₁-C₄)-alkyl, (C₁-C₄)-alkoxy, hydroxyl, halogen, trifluoromethyl, nitro, methylenedioxy, hydroxycarbonyl, (C₁-C₄)-alkoxycarbonyl, aminocarbonyl, cyano, phenyl, phenoxy and benzyloxy;
R¹⁰ represents hydroxyl or (C₁-C₈)-alkoxy, in particular (C₁-C₄)-alkoxy, and R¹⁰ preferably represents a radical from the group hydroxyl, methoxy, ethoxy, propoxy and isopropoxy;
R¹³ represents (C₁-C₆)-alkyl, (C₃-C₇)-cycloalkyl or benzyl, in particular methyl;
h represents 1 or 2, and preferably represents 1;
and the physiologically tolerated salts thereof.

6. 5-Membered ring heterocycles of the general formula I according to one or more of Claims 1, 4 and 5, in which, at the same time,
A represents a 1,4-phenylene radical;
R represents hydrogen;
R⁰ represents hydrogen;
R¹ represents an amino-imino-methyl radical;
R³ represents phenyl;
R¹⁰ represents hydroxyl, methoxy, ethoxy or isopropoxy;
R¹³ represents (C₁-C₆)-alkyl, (C₃-C₇)-cycloalkyl or benzyl, in particular methyl;
h represents 1,
and, preferably, a uniform configuration is in each case present at the chiral centre in the 4 position of the imidazolidine ring and at the chiral carbon atom carrying the radical R³;
and the physiologically tolerated salts thereof.

7. 5-Membered ring heterocycles of the general formula I according to one or more of Claims 1, 4, 5 and 6, in which, at the same time,
A represents a 1,4-phenylene radical;
R represents hydrogen;
R⁰ represents hydrogen;
R¹ represents an amino-imino-methyl radical;
R³ represents phenyl;
R¹⁰ represents hydroxyl, methoxy, ethoxy or isopropoxy;
R¹³ represents methyl;
h represents 1,
and, preferably, a uniform configuration is in each case present at the chiral centre in the 4 position of the imidazolidine ring and at the chiral carbon atom carrying the radical R³;
and the physiologically tolerated salts thereof.

8. 5-Membered ring heterocycles of the general formula I according to Claim 7, in which R¹⁰ represents hydroxyl, the S configuration is present at the chiral centre in the 4 position of the imidazolidine ring and the S configuration is present at the chiral carbon atom carrying the radical R³, and the physiologically tolerated salts thereof.

9. 5-Membered ring heterocycles of the general formula I according to Claim 7, in which R¹⁰ represents hydroxyl, the R configuration is present at the chiral centre in the 4 position of the imidazolidine ring and the S configuration is present at the chiral carbon atom carrying the radical R³, and the physiologically tolerated salts thereof.

10. 5-Membered ring heterocycles of the general formula I according to Claim 7, in which R¹⁰ represents ethoxy, the S configuration is present at the chiral centre in the 4 position of the imidazolidine ring and the S configuration is present at the chiral carbon atom carrying the radical R³, and the physiologically tolerated salts thereof.

11. 5-Membered ring heterocycles of the general formula I according to Claim 7, in which R¹⁰ represents ethoxy, the R configuration is present at the chiral centre in the 4 position of the imidazolidine ring and the S configuration is present at the chiral carbon atom carrying the radical R³, and the physiologically tolerated salts thereof.

12. Process for preparing compounds of the general formula I according to one or more of Claims 1 to 11, **characterized in that** a fragment condensation of a compound of the general formula II 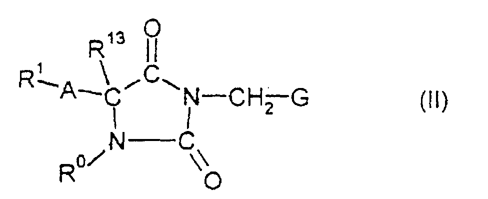 is carried out with a compound of the general formula III 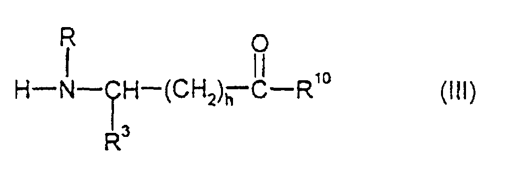 in which A, R, R⁰, R¹, R³, R¹⁰ and R¹³, and also h, are defined as indicated in Claims 1 to 11, and G represents hydroxycarbonyl, (C₁-C₆)-alkoxycarbonyl, activated carboxylic acid derivatives, such as acid chlorides, active esters or mixed anhydrides.

13. 5-Membered ring heterocycles of the general formula I according to one or more of Claims 1 to 11 and the physiologically tolerated salts thereof for use as medicaments.

14. 5-Membered ring heterocycles of the general formula I according to one or more of Claims 1 to 11, and the physiologically tolerated salts thereof, as inhibitors of thrombocyte aggregation, of the metastasization of carcinoma cells, or of the binding of osteoclasts to the bone surface.

15. 5-Membered ring heterocycles of the general formula I according to one or more of Claims 1 to 11, and the physiologically tolerated salts thereof, for treating arterial, venous or microcirculatory vascular diseases or thrombosis or for use when there is a risk of thrombosis.

16. Pharmaceutical preparation, **characterized in that** it contains one or more compounds of the general formula I according to one or more of Claims 1 to 11, and/or one or more physiologically tolerated salts thereof, as active compound together with pharmaceutically acceptable excipients and additives and, where appropriate, one or more different pharmacological active compounds as well.

17. Process for producing a pharmaceutical preparation, containing one or more compounds of the general formula I according to one or more of Claims 1 to 11, and/or one or more physiologically tolerated salts thereof, **characterized in that** these are brought, together with pharmaceutically acceptable excipients and additives and, where appropriate, one or more different pharmacological active compounds as well, into a suitable form for administration.

## Revendications

1. Hétérocycles à 5 chaînons de formule générale I 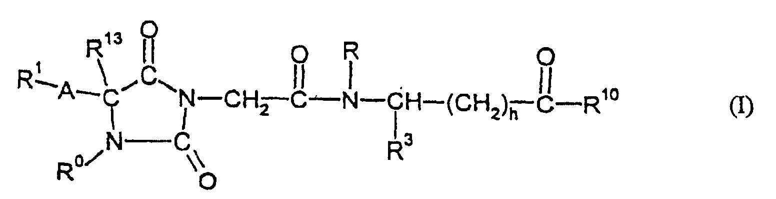 dans laquelle, en même temps,
A représente un reste 1,4-phénylène;
R et R⁰ représentent, indépendamment l'un de l'autre, un atome d'hydrogène ou un reste alkyle en C₁-C₄;
R¹ représente un reste H₂N-C(=NH), H₂N-C(=NH)-NH ou H₂N-CH₂;
R³ représente le reste CONHR¹⁴, le reste CONHR¹⁵ ou un reste phényle ou naphtyle non substitué, un reste phényle ou naphtyle substitué par un, deux ou trois restes identiques ou différents choisis dans le groupe constitué par les restes alkyle en C₁-C₄, alcoxy en C₁-C₄, hydroxy, halogéno, trifluorométhyle, nitro, méthylènedioxy, hydroxycarbonyle, (alcoxy en C₁-C₄)carbonyle, aminocarbonyle, cyano, phényle, phénoxy et benzyloxy, un reste pyridyle, un reste alkyle en C₁-C₄, un reste alcényle en C₂-C₄, un reste alcynyle en C₂-C₄ ou un reste cycloalkyle en C₅-C₆;
R¹⁰ représente un reste hydroxy ou alcoxy en C₁-C₈;
R¹³ représente un reste alkyle en C₁-C₆, cycloalkyle en C₃-C₇ ou benzyle;
R¹⁴ représente un reste méthyle substitué par phényle et hydroxycarbonyle, ou un reste méthyle substitué par phényle et (alcoxy en C₁-C₈)carbonyle;
R¹⁵ représente un reste adamantyle ou un reste adamantylméthyle;
h représente 1 ou 2;
et leurs sels physiologiquement acceptables.

2. Hétérocycles à 5 chaînons de formule générale I selon la revendication 1, dans lesquels, en même temps,
A représente un reste 1,4-phénylène;
R et R⁰ reorésentent, indépendamment l'un de l'autre, un atome d'hydrogène ou un reste alkyle en C₁-C₄, en particulier un atome d'hydrogène ou un reste méthyle ou éthyle;
R¹ représente un reste H₂N-C(=NH), H₂N-C(=NH)-NH ou H₂N-CH₂;
R³ représente le reste CONHR¹⁴;
R¹⁰ représente un reste hydroxy ou alcoxy en C₁-C₈, de préférence alcoxy en C₁-C₄;
R¹³ représente un reste alkyle en C₁-C₆, cycloalkyle en C₃-C₇ ou benzyle, en particulier un reste méthyle;
R¹⁴ représente un reste méthyle substitué par phényle et hydroxycarbonyle, ou un reste méthyle substitué par phényle et (alcoxy en C₁-C₈)carbonyle, de préférence (alcoxy en C₁-C₄)carbonyle;
h représente 1 ou 2, de préférence 1;
et leurs sels physiologiquement acceptables.

3. Hétérocycles à 5 chaînons de formule générale I selon la revendication 1, dans lesquels, en même temps,
A représente un reste 1,4-phénylène;
R et R⁰ représentent, indépendamment l'un de l'autre, un atome d'hydrogène ou un reste alkyle en C₁-C₄, en particulier un atome d'hydrogène ou un reste méthyle ou éthyle;
R¹ représente un reste H₂N-C(=NH), H₂N-C(=NH)-NH ou H₂N-CH₂;
R³ représente le reste CONHR¹⁵;
R¹⁰ représente un reste hydroxy ou alcoxy en C₁-C₈, de préférence alcoxy en C₁-C₄;
R¹³ représente un reste alkyle en C₁-C₆, cycloalkyle en C₃-C₇ ou benzyle, en particulier un reste méthyle;
R¹⁵ représente un reste adamantyle ou un reste adamantylméthyle;
h représente 1 ou 2, de préférence 1;
et leurs sels physiologiquement acceptables.

4. Hétérocycles à 5 chaînons de formule générale I selon la revendication 1, dans lesquels, en même temps,
A représente un reste 1,4-phénylène;
R et R⁰ représentent, indépendamment l'un de l'autre, un atome d'hydrogène ou un reste alkyle en C₁-C₄, en particulier un atome d'hydrogène ou un reste méthyle ou éthyle;
R¹ représente un reste H₂N-C(=NH), H₂N-C(=NH)-NH ou H₂N-CH₂;
R³ représente un reste phényle ou naphtyle non substitué, un reste phényle ou naphtyle substitué par un, deux ou trois restes identiques ou différents choisis dans le groupe constitué par les restes alkyle en C₁-C₄, alcoxy en C₁-C₄, hydroxy, halogéno, trifluorométhyle, nitro, méthylènedioxy, hydroxycarbonyle, (alcoxy en C₁-C₄)carbonyle, aminocarbonyle, cyano, phényle, phénoxy et benzyloxy, un reste pyridyle, un reste alkyle en C₁-C₄, un reste alcényle en C₂-C₄, un reste alcynyle en C₂-C₄ ou un reste cycloalkyle en C₅-C₆, et R³ représente en particulier un reste phényle;
R¹⁰ représente un reste hydroxy ou alcoxy en C₁-C₈, en particulier alcoxy en C₁-C₄, et R¹⁰ représente de préférence un reste choisi dans le groupe constitué par les restes hydroxy, méthoxy, éthoxy, propoxy et isopropoxy;
R¹³ représente un reste alkyle en C₁-C₆, cycloalkyle en C₃-C₇ ou benzyle, en particulier un reste méthyle;
h représente 1 ou 2, de préférence 1;
et leurs sels physiologiquement acceptables.

5. Hétérocycles à 5 chaînons de formule générale I selon les revendications 1 et/ou 4, dans lesquels, en même temps,
A représente un reste 1,4-phénylène;
R et R⁰ représentent, indépendamment l'un de l'autre, un atome d'hydrogène ou un reste alkyle en C₁-C₄, en particulier un atome d'hydrogène ou un reste méthyle ou éthyle;
R¹ représente un reste H₂N-C(=NH), H₂N-C(=NH)-NH ou H₂N-CH₂;
R³ représente un reste phényle non substitué ou un reste phényle substitué par un, deux ou trois restes identiques ou différents choisis dans le groupe constitué par les restes alkyle en C₁-C₄, alcoxy en C₁-C₄, hydroxy, halogéno, trifluorométhyle, nitro, méthylènedioxy, hydroxycarbonyle, (alcoxy en C₁-C₄)carbonyle, aminocarbonyle, cyano, phényle, phénoxy et benzyloxy;
R¹⁰ représente un reste hydroxy ou alcoxy en C₁-C₈, en particulier alcoxy en C₁-C₄, et R¹⁰ représente de préférence un reste choisi dans le groupe constitué par les restes hydroxy, méthoxy, éthoxy, propoxy et isopropoxy;
R¹³ représente un reste alkyle en C₁-C₆, cycloalkyle en C₃-C₇ ou benzyle, en particulier un reste méthyle;
h représente 1 ou 2, de préférence 1;
et leurs sels physiologiquement acceptables.

6. Hétérocycles à 5 chaînons de formule générale I selon l'une ou plusieurs des revendications 1, 4 et 5, dans lesquels, en même temps,
A représente un reste 1,4-phénylène;
R représente un atome d'hydrogène;
R⁰ représente un atome d'hydrogène;
R¹ représente un reste amino-iminométhyle:
R³ représente un reste phényle;
R¹⁰ représente un reste hydroxy, méthoxy, éthoxy ou isopropoxy;
R¹³ représente un reste alkyle en C₁-C₆, cycloalkyle en C₃-C₇ ou benzyle, en particulier un reste méthyle;
h représente 1;
et, de préférence, au niveau du centre de chiralité en position 4 du cycle imidazolidine et au niveau de l'atome de carbone chiral portant le reste R³, il y a à chaque fois une configuration unique;
et leurs sels physiologiquement acceptables.

7. Hétérocycles à 5 chaînons de formule générale I selon l'une ou plusieurs des revendications 1, 4, 5 et 6, dans lesquels, en même temps,
A représente un reste 1,4-phénylène;
R représente un atome d'hydrogène;
R⁰ représente un atome d'hydrogène;
R¹ représente un reste amino-iminométhyle;
R³ représente un reste phényle;
R¹⁰ représente un reste hydroxy, méthoxy, éthoxy ou isopropoxy;
R¹³ représente un reste méthyle;
h représente 1;
et, de préférence, au niveau du centre de chiralité en position 4 du cycle imidazolidine et au niveau de l'atome de carbone chiral portant le reste R³, il y a à chaque fois une configuration unique;
et leurs sels physiologiquement acceptables.

8. Hétérocycles à 5 chaînons de formule générale I selon la revendication 7, dans lesquels R¹⁰ représente le reste hydroxy, le centre de chiralité en position 4 du cycle imidazolidine présente la configuration S et l'atome de carbone chiral portant le reste R³ présente la configuration S, et leurs sels physiologiquement acceptables.

9. Hétérocycles à 5 chaînons de formule générale I selon la revendication 7, dans lesquels R¹⁰ représente le reste hydroxy, le centre de chiralité en position 4 du cycle imidazolidine présente la configuration R et l'atome de carbone chiral portant le reste R³ présente la configuration S, et leurs sels physiologiquement acceptables.

10. Hétérocycles à 5 chaînons de formule générale I selon la revendication 7, dans lesquels R¹⁰ représente le reste éthoxy, le centre de chiralité en position 4 du cycle imidazolidine présente la configuration S et l'atome de carbone chiral portant le reste R³ présente la configuration S, et leurs sels physiologiquement acceptables.

11. Hétérocycles à 5 chaînons de formule générale I selon la revendication 7, dans lesquels R¹⁰ représente le reste éthoxy, le centre de chiralité en position 4 du cycle imidazolidine présente la configuration R et l'atome de carbone chiral portant le reste R³ présente la configuration S, et leurs sels physiologiquement acceptables.

12. Procédé de préparation de composés de formule générale I selon l'une ou plusieurs des revendications 1 à 11, **caractérisé en ce que** l'on effectue une condensation de fragments d'un composé de formule générale II 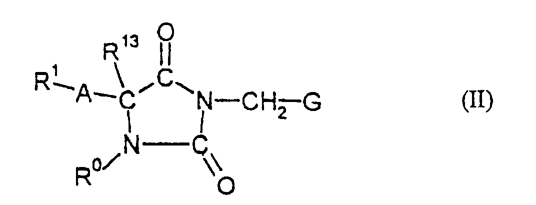 avec un composé de formule générale III 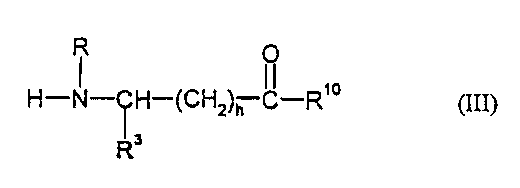 où A, R, R⁰, R¹, R³, R¹⁰ et R¹³, ainsi que h, ont la définition indiquée dans les revendications 1 à 11, et G représente un reste hydroxycarbonyle, (alcoxy en C₁-C₆)carbonyle, un dérivé activé d'acide carboxylique comme un chlorure d'acide, un ester actif ou un anhydride mixte.

13. Hétérocycles à 5 chaînons de formule générale I selon l'une ou plusieurs des revendications 1 à 11 et leurs sels physiologiquement acceptables pour une application comme médicaments.

14. Hétérocycles à 5 chaînons de formule générale I selon l'une ou plusieurs des revendications 1 à 11 et leurs sels physiologiquement acceptables comme substances inhibant l'agrégation des thrombocytes, la formation de métastases de cellules cancéreuses ou la fixation d'ostéoclastes sur la surface des os.

15. Hétérocycles à 5 chaînons de formule générale I selon l'une ou plusieurs des revendications 1 à 11 et leurs sels physiologiquement acceptables pour le traitement de maladies vasculaires artérielles, veineuses ou microcirculatoires, de thromboses ou du risque de thrombose.

16. Composition pharmaceutique, **caractérisée en ce qu'**elle contient comme substance active un ou plusieurs composés de formule générale I selon l'une ou plusieurs des revendications 1 à 11 et/ou un ou plusieurs de leurs sels physiologiquement acceptables avec des supports et des additifs pharmaceutiquement acceptables et éventuellement une ou plusieurs autres substances actives pharmacologiques.

17. Procédé de préparation d'une composition pharmaceutique contenant un ou plusieurs composés de formule générale I selon l'une ou plusieurs des revendications 1 à 11 et/ou un ou plusieurs de leurs sels physiologiquement acceptables, **caractérisé en ce que** l'on met ces composés sous une forme d'administration appropriée avec des supports et des additifs pharmaceutiquement acceptables et éventuellement une ou plusieurs autres substances actives pharmacologiques.
